(19) Europäisches Patentamt
European Patent Office
Office européen des brevets

(11) **EP 3 226 200 A1**

(12) **EUROPEAN PATENT APPLICATION**
published in accordance with Art. 153(4) EPC

(43) Date of publication:
04.10.2017 Bulletin 2017/40

(21) Application number: 15862973.3

(22) Date of filing: 25.11.2015

(51) Int Cl.:
*G06Q 50/22* (2012.01)  *A61B 10/00* (2006.01)
*G06Q 50/10* (2012.01)

(86) International application number:
**PCT/JP2015/083035**

(87) International publication number:
**WO 2016/084834 (02.06.2016 Gazette 2016/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: 25.11.2014 JP 2014238283

(71) Applicant: **Hitachi High-Technologies Corporation**
**Tokyo 105-8717 (JP)**

(72) Inventors:
• **HASEGAWA, Kiyoshi**
  **Tokyo 105-8717 (JP)**
• **NASU, Kiyoshi**
  **Yokohama-shi**
  **Kanagawa 244-0817 (JP)**
• **TANAKA, Shigeya**
  **Hitachinaka-shi**
  **Ibaraki 312-8504 (JP)**
• **ISHIZUKA, Toshihiro**
  **Tokyo 105-8717 (JP)**

(74) Representative: **Strehl Schübel-Hopf & Partner**
**Maximilianstrasse 54**
**80538 München (DE)**

(54) **MEASUREMENT SYSTEM, HEAD-MOUNTED DEVICE, PROGRAM, AND SERVICE PROVIDING METHOD**

(57) A head mount apparatus mounted on a head of a user is disclosed, which includes: detection means to detect a variation of a bloodflow rate of the head; and transfer means to transfer a detection value of the detection means to a predetermined transfer destination.

An information processing apparatus is also disclosed, which includes; receiving means to receive a detection value transferred from transfer means; and service providing means to provide a user with a service based on the received detection value.

*FIG. 1*

EP 3 226 200 A1

**Description**

TECHNICAL FIELD

The present invention pertains to a measurement system, a head-mounted device, a program, and a service providing method.

BACKGROUND ART

A measurement system has hitherto been provided, which acquires information representing activity states of a brain by providing a near infrared ray irradiation unit and an near infrared ray detection unit on a head mount apparatus (a head-mounted device) called a headset, detecting a variation of a bloodflow rate of a brain surface, and causing a data processing apparatus to process detected data.

[Documents of Prior Arts]

[Patent Documents]

[Patent Document 1] Japanese Patent Application Laid-Open Publication No. 2006-320735

[Non-Patent Documents]

[Non-Patent Document 1] "Brain Science, Now and Here; Forefront of Solution Business Enabled by Visualization Technology of Brain Functions", [online], Nikkei Shimbun (Japan Economic Newspaper), [Searched on Nov. 17, 2014], Internet <http://ps.nikkei.co.jp/hightech/v9-01.html>
[Non-Patent Document 2] "Practical Use of Optical Topographical Technology", [online] Hitachi High-Technologies Corp, [Searched on Nov. 17, 2014], Internet <http://www.hitachi.co.jp/products/ot/hardware/wot.html>

SUMMARY OF THE INVENTION

[Problems to be solved by the Invention]

However, the conventional measurement system is configured to include a dedicated data processing apparatus for processing detected data, in which there are a limitation to a data processing function to be provided and a limitation to applications of processed data. Therefore, a general user has a problem against easily using the measurement system. As a result, the conventional measurement system does not reach a point of being utilized by a multiplicity of users or effectively broadly used in a variety of phases of society.

Under such circumstances, it is an object of the present invention to provide a technology enabled to simply acquire a variation of a bloodflow rate of a head and to be broadly effectively used.

[Means for solving the Problems]

One aspect of the present invention can be exemplified by a measurement system that follows. The present measurement system includes a head mount apparatus and an information processing apparatus. The head mount apparatus includes: detection means to detect a variation of a bloodflow rate of a head of a user, the detection means being mounted on the head; and transfer means to transfer a detection value of the detection means to a predetermined transfer destination, The information processing apparatus includes : receiving means to receive the detection value transferred from the transfer means; and service providing means to provide a service to the user, based on the received detection value.

A second aspect of the present invention can be exemplified by a head mount apparatus including: means to have marks used for alignment with a reference position of the head when mounted on the head of the user; detection means to detect a variation of a bloodflow rate of the head in a state of being already aligned with the reference position; and transfer means to transfer a detection value of the detection means to a predetermined transfer destination.

A third aspect of the present invention can be exemplified by a program for making a computer execute: a receiving step of receiving a detection value transferred from a head mount apparatus mounted on a head of a user and detecting a variation of a bloodflow rate of the head; and a service providing step of providing a service to the user, based on the received detection value.

A fourth aspect of the present invention can be exemplified by a program for making a computer execute: a step of accepting a request for providing a fee-charging service based on a detection value of a variation of a bloodflow rate of a head, the variation being detected by a head mount apparatus mounted on the head of a user; a step of instructing a server on a network to execute an accounting process for the fee-charging service upon accepting the fee-charging service providing request; and a service providing step of providing the user with the fee-charging service after completing the accounting process.

A fifth aspect of the present invention can be exemplified by a service providing method by which a computer executes: a step of accepting a request for providing a fee-charging service based on a detection value of a variation of a bloodflow rate of a head from an information processing apparatus of a user, the variation being detected by a head mount apparatus mounted on the head of the user; a step of instructing an accounting server on a network to execute an accounting process for the fee-charging service upon accepting the fee-charging service providing request from the information processing apparatus; a step of acquiring the detection value from the information processing apparatus; and a service providing step of providing the fee-charging service.

A further aspect of the present invention can be exemplified by a service providing method by which a computer executes: a step of accepting, from an information processing apparatus of a user, a request for a fee-charging download of an application program for processing a detection value of a variation of a bloodflow rate of a head, the variation being detected by a head mount apparatus mounted on the head of the user; a step of transmitting, to an accounting server on a network, execution of an accounting process about the application program upon accepting the fee-charging download request from the information processing apparatus; and a step of transmitting the application program to the information processing apparatus.

[Effect of the Invention]

The present invention provides the technology enabled to simply acquire the variation of the bloodflow rate of the head and to be broadly effectively used.

BRIEF DESCRIPTION OF THE DRAWINGS

FIG. 1 is a diagram illustrating a configuration participating in information processing of a measurement system according to one embodiment.
FIG. 2 is a perspective view illustrating an external appearance of a head mount apparatus.
FIG. 3 is a plan view of the head mount apparatus as viewed from upward.
FIG. 4 is a front view of the head mount apparatus as viewed from a front.
FIG. 5 is a diagram illustrating an example of a screen displayed by a user terminal when performing calibration of the first time.
FIG. 6 is a diagram illustrating a process of the calibration of the second time onward.
FIG. 7 is a view illustrating a model coordinate system of a user in an Example 1.
FIG. 8 is a diagram illustrating an example of a measurement position management table.
FIG. 9 is a diagram of an example of a structure management table.
FIG. 10 is a diagram of an example of a sensor slider set value management table.
FIG. 11 is a diagram of an example of data retained on a memory 22 by the user terminal.
FIG. 12 is a flowchart illustrating an alignment processing procedure of the first time.
FIG. 13 is a flowchart illustrating details of a process of an image processing unit.
FIG. 14 is a flowchart illustrating details of a process of a position computing unit.

FIG. 15 is a flowchart illustrating details of a process of an image synthesizing unit.
FIG. 16 is a diagram illustrating a process of the calibration from the second time onward.
FIG. 17 is a flowchart illustrating the process of the calibration from the second time onward.
FIG. 18 is a diagram illustrating a configuration of the measurement system in an Example 2.
FIG. 19 is a diagram illustrating a configuration of the measurement system in an Example 3.
FIG. 20 is a diagram illustrating an operational example of the user terminal.
FIG. 21 is a diagram illustrating an operational example of the user terminal.

EMBODIMENTS

[Mode for Carrying out the Invention]

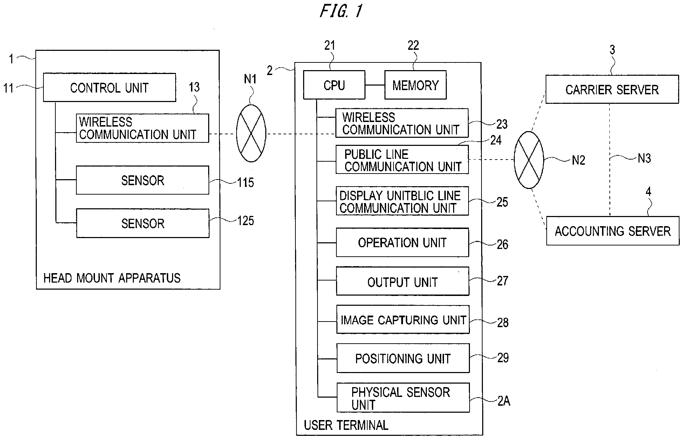

A measurement system according to one embodiment will hereinafter be described with reference to the drawings. FIG. 1 is a diagram illustrating a configuration participating in information processing of the measurement system according to one embodiment of the present invention. The measurement system detects measurement data (which is also termed a detection value) representing a variation of a bloodflow rate from a head of a user, and acquires brain activity information indicating an activity state of a brain of the user. The measurement system provides the user a variety of services, based on the acquired brain activity information.

<Example of System Architecture>

As in FIG. 1, the measurement system includes a head mount apparatus 1 and a user terminal 2. The headmount apparatus 1 includes, as an aspect of the information processing, a control unit 11, a wireless communication unit 13, and a couple of sensors 115, 125. The control unit 11 controls measurement and communications of the head mount apparatus 1. The control unit 11 includes a processor instanced by a CPU (Central Processing Unit) or a DSP (Digital Signal Processor) and a memory, and executes processing based on a computer program, firmware and other equivalent software that are deployed in an executable manner on the memory. However, the control unit 11 may be a dedicated hardware circuit, an FPGA (Field Programmable Gate Array) and other equivalent circuits, which execute cooperative processing with respective components by starting up the wireless communication unit 13 and the sensors 115, 125. The control unit 11 may also have a coexisting configuration of the CPU, the DSP, and the dedicated hardware circuit.

The wireless communication unit 13 is connected to the control unit 11 and the sensors 115, 125 via a predetermined interface. The wireless communication unit 13 may also be, however, configured to acquire the data from the sensors 115, 125 via the control unit 11. The wireless communication unit 13 performs communications with the user terminal 2 via a network N1. The network N1 is a network conforming to standards exemplified by Bluetooth (registered trademark), a wireless LAN (Local Area Network) and ZigBee. The wireless communication unit 13 is one example of "transfer means". It does not, however, mean that the interface is limited to the standards of the wireless interface of the wireless communication unit 13 in the present measurement system.

The measurement system may be provided with a communication unit performing wired communications in place of the wireless communication unit 13 or together with the wireless communication unit 13. In other words, the head mount apparatus 1 and the user terminal 2 may be interconnected via an interface for the wired communications. It does not mean that the interface is limited to the interface for the wired communications in this case, but a variety of interfaces exemplified by a USB (Universal Serial Bus) and a PCI Express are usable corresponding to applications of the measurement system.

Each of the sensors 115, 125 irradiates the head with near infrared rays, receives the near infrared rays partly absorbed but scattered in the vicinity of a cerebral cortex of the brain, and converts the received near infrared rays into electrical signals. The cerebral cortex of the brain has different bloodflow rates corresponding to, e.g., activity states of the brain. As a result, a quantity of hemoglobin bound to oxygen in the blood and a quantity of the hemoglobin not bound to the oxygen vary in respective regions of the cerebral cortex. An absorptive characteristic or a scattering characteristic of the near infrared rays in the vicinity of the cerebral cortex varies due to variations of a hemoglobin quantity and an oxygen quantity. Each of the sensors 115, 125 converts the near infrared rays, of which a light quantity varies due to a variation of an absorption ratio or a transmittance of the near infrared rays corresponding to a state of the bloodflow in the vicinity of the cerebral cortex, into the electrical signals and outputs the electrical signals. The sensors 115, 125 are one example of "detection means".

Each of the sensors 115, 125 includes a source of near infrared rays to irradiate the near infrared rays, and a light receiving unit to receive the near infrared rays. The source of near infrared rays is exemplified by an LED (Light Emitting Diode) and an infrared-ray lamp. The light receiving unit includes a photo-electric element instanced by a photo diode and a photo transistor, an amplifier and an AD (Analog Digital) converter. Note that the source of near infrared rays and the light receiving unit may not be provided in pairs. For example, a plurality of light receiving units may also be provided for one source of near infrared rays.

The user terminal 2 is exemplified by a mobile phone, a PDA (Personal Digital Assistant), a PHS (Personal Handy Phone System), and a portable personal computer. However, the user terminal 2 may also be, depending on functions of applications, a non-portable desktop personal computer, a TV receiver, a game machine, a terminal dedicated to health management, a massage machine, and an on-vehicle equipment.

The user terminal 2 acquires, from the head mount apparatus 1, variation data of the absorption ratio or the transmittance of the near infrared rays in the vicinity of the cerebral cortex of the user, and provides services including various types of information processing pertaining to the brain activity states of the user.

The user terminal 2 includes a CPU 21, a memory 22, a wireless communication unit 23, a public line communication unit 24, a display unit 25, an operation unit 26, an output unit 27, an image capturing unit 28, a positioning unit 29, and a physical sensor unit 2A. The CPU 21 executes a process as the user terminal 2, based on a computer program deployed in the executable manner on the memory 22. The process as the user terminal 2 is defined as, e.g., a service containing a variety of information processes pertaining to the brain activity states of the user. The CPU 21 running the computer program is one example of "service providing means".

The memory 22 stores the computer program to be run by the CPU 21 or data to be processed by the CPU 21. The memory 22 may include a volatile memory and a non-volatile memory. The wireless communication unit 23 is the same as the wireless communication unit 13 of the head mount apparatus 1. The wireless communication unit 23 is one example of "receiving means". The user terminal 2 may include a communication unit to perform wired communications in place of the wireless communication unit 13 or together with the wireless communication unit 13.

The public line communication unit 24 performs the communications with a sever on a network N2, e.g., a carrier server 3 via the network N2. The network N2 is a public line network, e.g., a mobile phone network. When the network N2 is the mobile phone network, the public line communication unit 24 connects to the network N2 via a base station of the mobile phone network. However, the network N2 may also be a network including an access network to communication equipments of Internet providers, and the Internet. The access network to the communication equipments of the Internet providers is exemplified by an optical network and ADSL (Asymmetric Digital Subscriber Line) provided by the carriers. The network N2 is one example of "public wireless communication means". It does not, however, mean that the network N2 is limited to the public line network in the present measurement system, but the network N2 may also be an in-house network instanced by a LAN (Local Area Network), a dedicated line of an enterprise, an entrepreneur, a city hall, a school and a research institute, and a wide area network instanced by VPN (Virtual Private Network). The enterprise, the entrepreneur, the city hall, the school and the research institute will hereinafter be simply referred to also as the enterprise and other equivalent organizations.

The display unit 25 is instanced by a liquid crystal display and an EL (Electro-Luminescence) panel, and displays information outputted from the CPU 21. The operation unit 26 is exemplified by a push button and a touch panel, and accepts a user's operation. The output unit 27 is exemplified by a vibrator to output vibrations and a loudspeaker to output sounds or voices. The image capturing unit 28 is exemplified by a camera including a solid-state image sensing device. The solid-state image sensing device can involve using a CCD (Charged-Coupled Device) image sensor, a CMOS (Complementary Metal Oxide Semiconductor) image sensor and other equivalent image sensors. The positioning unit 29, which is, e.g., a GPS (Global Positioning System) receiver, receives radio waves from a GPS satellite and computes a present position (latitude, longitude and other equivalent coordinates) and the time. It does not, however, mean that the positioning unit 29 is limited to a unit including the GPS receiver. For example, the public line communication unit 24 is the mobile phone network, in which case the positioning unit 29 may execute measuring a position based on a distance from the base station of the mobile phone. The physical sensor unit 2A is exemplified by an acceleration sensor or an angular acceleration sensor. The physical sensor unit 2A may also, however, be a temperature sensor, a humidity sensor, an air pressure sensor or a water pressure sensor.

The carrier server 3 and an accounting server 4 are interconnected via the network N2 or a dedicated network N3. The dedicated network N3 is instanced by a network connected to computers of a financial institute, a dedicated network of the enterprise, and the VPN.

<Example of Structure of Head Mount Apparatus>

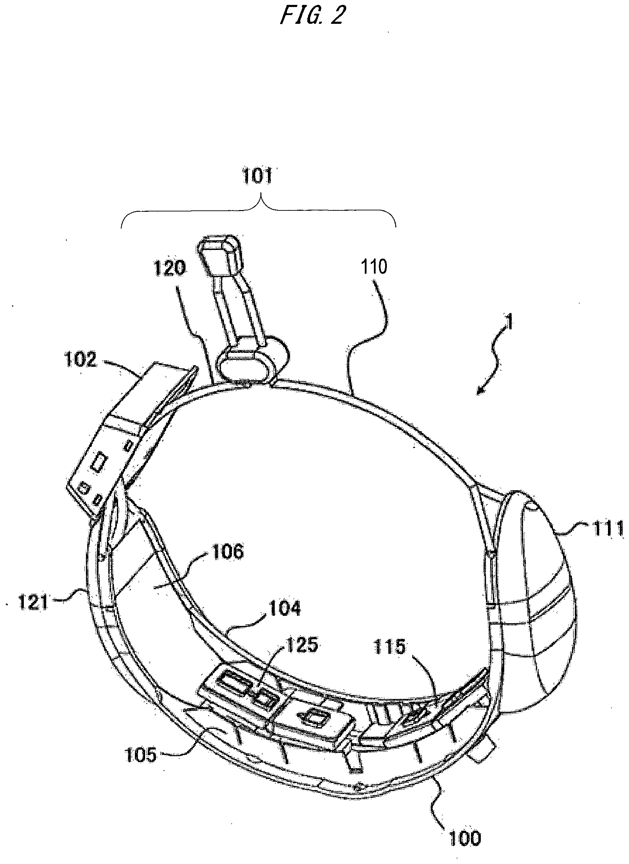

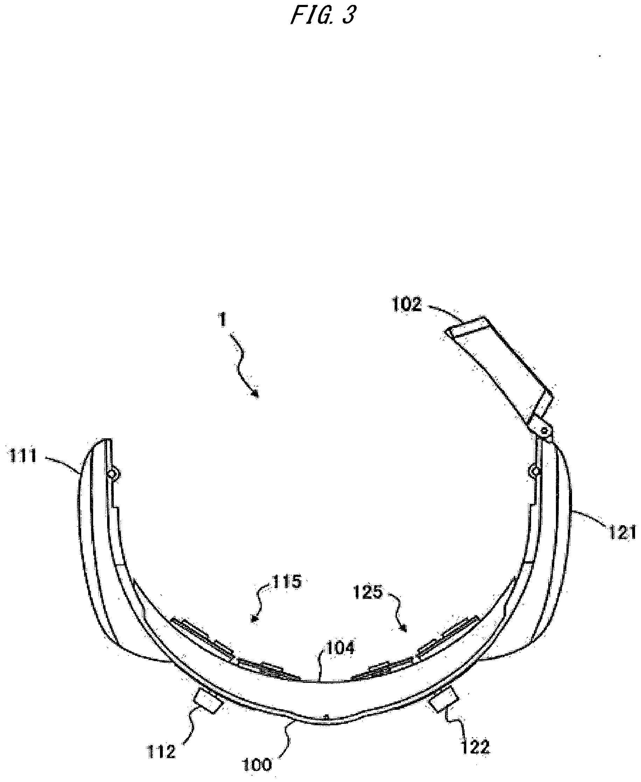

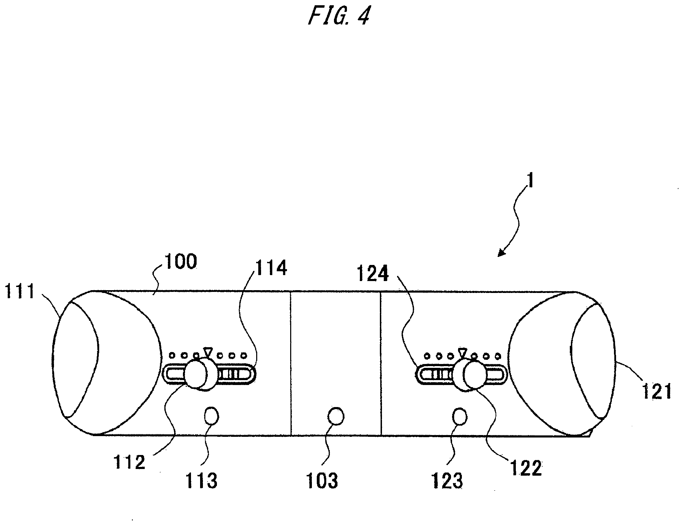

FIG. 2 is a perspective view of an external appearance of the head mount apparatus 1 as viewed from a bottom on a rear side. FIG. 3 is a plan view of the head mount apparatus 1 as viewed from upward. FIG. 4 is a front view of the head mount apparatus 1 as viewed from a front. However, FIGS. 3 and 4 omit an illustration of a fixation member 101 depicted in FIG. 2. Herein, "the bottom on the rear side" indicates a rear side of the user and a position of looking up the user's head from the bottom when the user wears the head mount apparatus 1. Further, "the front" indicates the front of the user (the wearer) when the user wears the head mount apparatus 1. The term "upward" indicates, e.g., an upper region of the user. Note that a portion of the head mount apparatus 1 positioned on the right side when directed to the user wearing the head mount apparatus 1 is termed a right-side portion or simply a right side. A portion of the headmount apparatus 1 positioned on the left side when directed to the user wearing the head mount apparatus 1 is termed a left-side portion or simply a left side. A face of the head mount apparatus 1 contacting the user is termed a rear face. A face opposite to the rear face is termed a front face. The front face is a face visible from a periphery of the user when the user wears the head mount apparatus 1.

As illustrated in FIG. 2, the head mount apparatus 1 has such a structure that the head mount apparatus 1 is wound around the head in a headband shape and fixed to the user's head by fastening the fixation member 101. The head mount apparatus 1 therefore includes a belt-shaped base member 100 bent in a slightly larger space than a human head, and the fixation member 101 fixed to both ends of the base member 100. The fixation member 101 includes wire members 110, 120 extending from the both ends of the base member 100, and fasteners for fixing the wire members 110, 120 by withdrawing the couple of wire members 110, 120. The base member 100 forms an external face distanced from a surface of the user's head. To be specific, the head mount apparatus 1 is structured to dispose the base member 100 as a member retaining the shape on the external face distanced from the head. The base member 100 is composed of, e.g., a resin. It does not, however, mean that the base member 100 is limited to the material.

Note that it does not mean that the fixation member 101 is limited to a structure, a shape and a material in the embodiment. For example, in FIG. 2, the fixation member 101 has the wire members 110, 120 and may also use band-shaped members in place of the wire members. The fastener may take any structure.

As in FIG. 2, a battery box 102 is provided at an end portion, on the right side, of the base member 100 of the head mount apparatus 1. The battery box 102 takes substantially a flat hexahedron, in which an area size of each of the front face and the rear face is larger than a total area size of four side faces. An unillustrated groove is formed in the rear face of the battery box 102. A mid-portion of the wire member 120 extending from the end portion, on the right side, of the base member 100 is fitted in this groove. Accordingly, the battery box 102 is fixed to the end portion, on the right side, of the base member 100, and is, with the wire member 120 being fitted in the groove, thereby fixed to the head mount apparatus 1.

Two roundish housings 111, 121 are provided in the vicinities of both ends, on the front face side, of the base member 100 of the head mount apparatus 1. Each of the housings 111, 121 houses a control board including a signal processing circuit and a communication circuit. As in FIG. 4, when viewing the head mount apparatus 1 from the front face, the two housings 111, 121 appear to be positioned on both sides of the head mount apparatus 1.

As in FIG. 4, three markers 113, 103, 123 are so provided in bilateral symmetry with respect to the marker 103 being centered in the vicinity of the front face, on the front side, of the base member 100 as to be on a straight line in positions along a lower edge of the base member 100. The markers 113, 103, 123 may have any structure as far as being enabled to distinguish between positions of the markers 113, 103, 123 on images when captured by the image capturing unit 28. It is, however, desirable that the markers 113, 103, 123 are enabled to recognize their area sizes on the images when captured by the image capturing unit 28. For example, the markers 113, 103, 123 may be configured as recessed portions, provided in the base member 100, each having a bottom face taking a circular or polygonal shape. The markers 113, 103, 123 may also be configured as protruded portions, provided on the base member 100, each having a section taking the circular or polygonal shape. The markers 113, 103, 123 may also be formed by applying coating to the base member 100. The markers 113, 103, 123 may further be formed by applying the coating to the recessed portions or the protruded portions.

In the vicinity of the front face of the base member 100 on the front side, upper portions of the markers 113, 123 are formed with band-shaped apertures 114, 124, and knobs 112, 122 are inserted into the apertures 114, 124. The knobs 112, 122 are connected to unillustrated right and left sliders provided along the rear face of the base member 100. On the other hand, as depicted in FIG. 2, sensors 115, 125 are fixed to the sliders on the rear face of the base member 100. Accordingly, the knob 112 or the knob 122 is moved along the band-shaped aperture 114 or aperture 124 relatively to the base member 100, thereby enabling the sensor 115 or the sensor 125 to move on the rear face respectively. Screws are formed coaxially with the knobs 112, 122, whereby positions of the sensors can be fixed by a screw method. The knobs 112, 122 and the markers 113, 103, 123 are one example of "alignment means".

In FIG. 2, the knob takes a low cylindrical shape, but it does not mean that the knob is limited to its shape. In FIG. 2, scales are formed along longitudinal directions of the band-shaped apertures 114, 124. The scales take different shapes for distinguishing between the scale in a central position and the scales in other positions, thereby recognizing the central position. In FIG. 2, the scale in the central position takes a triangular shape with an apex being directed to the apertures 114, 124, while the scales in the positions other than the central position are formed in a circular or dotted shape. A method of forming the scales is the same as the method of forming the markers 113, 103, 123, but it does not mean that the scaling method is limited to a particular method.

As illustrated in FIG. 2, each of the sensors 115, 125 takes such a shape that the flat plates are formed with three windows. One of the windows of each of the sensors 115, 125 is provided with a near infrared ray LED as the near infrared ray source . The remaining two windows of each of the sensors 115, 125 are provided with photo diodes or photo transistors as the light receiving units.

Note that it does not mean that a number of the light receiving units of each of the sensors 115, 125 is limited to "2". For example, each of the sensors 115, 125 may be provided with one light receiving unit and may also be provided with three or more light receiving units.

For instance, each of the sensors 115, 125 is provided with the two light receiving units, and, when the respective light receiving units are discriminated as different sensors, these light receiving units are to be called sensors 115-1, 115-2, 125-1 and 125-2. In the present specification, however, the near infrared ray sources and the light receiving units are called as integral units like the sensors 115, 125.

Light shielding units 104, 105 are provided at upper and lower edges of the base member 100. Therefore, the sensors 115, 125 are installed in a space interposed between the light shielding units 104, 105 of the upper and lower edges on the rear face of the base member 100. The light shielding units 104, 105 also function as buffer members at portions contacting a forehead on the rear face of the head mount apparatus 1. It does not mean that the light shielding units 104, 105 are limited to their materials, but it is desirable that the light shielding units 104, 105 are composed of light and soft materials because of contact the user's head. The light shielding units 104, 105 are composed of the resin instanced by urethan, and a rubber.

A wire arrangement for connecting the battery box 102, the sensors 115, 125 and boards within the housings 111, 121 is made on the rear face of the base member 100. However, portions other than the portions, provided with the sensors 115, 125, of the base member 100 are covered with a cover 106. The cover 106 functions as a shielding member for preventing the boards and the wires from directly contacting a skin of the user on the rear face side of the head mount apparatus 1 contacting the head. Hence, the wires are arranged in a space between the base member 100 and the cover 106.

<Support for Aligning Sensors>

In the measurement system, the CPU 21 of the user terminal 2 supports the user for aligning the sensors 115, 125 in accordance with an alignment application program (which will hereinafter be simply termed the alignment application) deployed in the executable manner on the memory 22. A process that the user terminal 2 supports the user for aligning the sensors 115, 125, is also called calibration. Through the calibration, the user terminal 2 guides the user so that the sensors 115, 125 are disposed in desirable positions of the user's head. When the suitable calibration is conducted, it follows that the sensors 115, 125 detect the variation of the blood flow rate in the desirable positions of the user's head.

The positions of the user's head, which are desirable for the calibration targets, differ depending on various types of services, functions and applications utilized in the measurement system. For example, the user terminal 2 runs an application program (which will hereinafter be referred to as a brain application), thereby providing various types of services or functions by using the measurement data transmitted from the head mount apparatus 1. This being the case, it is desirable that the sensors 115, 125 are disposed at measurement regions per brain application before running the brain application.

In the calibration, the image capturing unit 28 captures an image of the user's head, and the user terminal 2 displays the captured image on the display unit 25. One example is that the user terminal 2 displays objects indicating present positions and target positions of the sensors 115, 125 by being superposed on the image of the user's head. The user terminal 2 supports the user so that the present positions of the sensors 115, 125 get close to the target positions. Another example is that the user terminal 2 may guide the user so that characteristic points, e.g., the positions of the markers 113, 103, 123 or the knobs 112, 122 of the headmount apparatus 1 are disposed in desirable positions with respect to the image of the user's head.

(1) Calibration of First Time

When the user utilizes the brain application for the first time, the calibration of the first time is carried out. The calibration of the first time is a process of supporting the user for an operation of aligning the sensors 115, 125 in the target positions of the user's head that are prescribed by the brain application when the user utilizes a certain brain application for the first time. However, the calibration of the first time is also said to be a process of generating a reference image used for the calibrations from the second time onward.

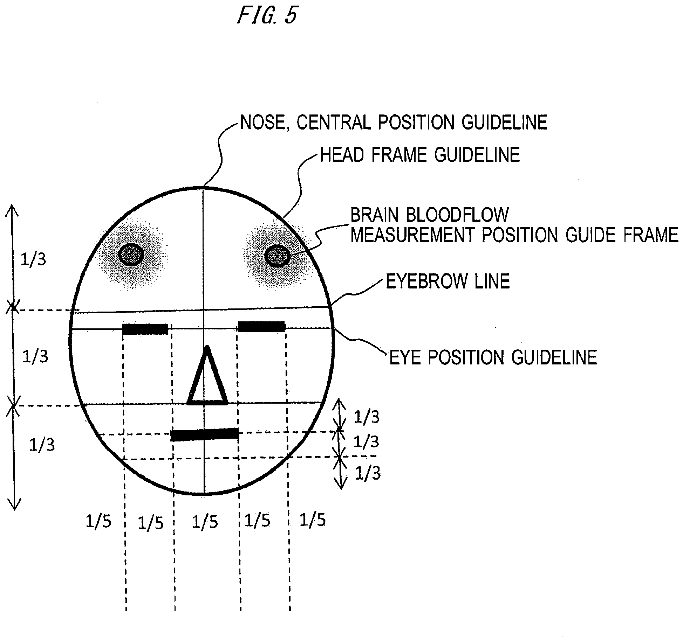

FIG. 5 illustrates a screen displayed by the user terminal 2 when performing the calibration of the first time. Displayed on the screen in FIG. 5 are a head frame guideline, a brain bloodflow measurement position guide frame, an eye position guideline and a nose/central position guideline by being superposed on the image of the user's head. The head frame guideline is, e.g., an outline surrounding an area having predetermined a dimension, which simulates the human head. It is desirable that the dimension of the head frame guideline is a size suitable for being displayed on the display unit 25 and is an image dimension sufficient not to cause any hindrance against supporting the user for the alignment. The eye position guidelines are, e.g., line segments passing through centers of the right and left eyes, respectively. The nose/central position guideline is, e.g., a vertical line segment connecting to a central line of the nose. The brain bloodflow measurement position guide frames are target positions in which the sensors 115, 125 are aligned.

In the calibration of the first time, the user terminal 2, at first, guides the user so that the head image of the user takes a desirable posture. To begin with, the user controls, e.g., a distance to the image capturing unit 28 of the user terminal 2, thereby making the outline of the user's head coincident with the head frame guideline. The user modifies the position of the head so that the eyes and the nose of the user are coincident with, e.g. , the eye position guideline and the nose/central position guideline.

Next, the user makes the alignment of the head mount apparatus 1. The user terminal 2 displays the marks indicating the present positions of the sensors 115, 125 in superposition on the image of the user's head on the basis of the shape and the dimension of the present head mount apparatus 1 and the positions of the knobs 112, 122 in the image of the user's head. The user adjusts a wearing state of the head mount apparatus 1 so that the present positions of the sensors 115, 125 are superposed on the target positions indicated by the brain bloodflow measurement position guide frames. Note that the user terminal 2 may instruct the user by a message and other equivalent notifications on the display unit 25 so that the positions of the knobs 112, 122 are set to predetermined knob positions (default positions) beforehand per brain application to be run by the user.

The brain bloodflow measurement position guide frames (target positions) may also be aligned with the positions of the sensors 115, 125 in a three-dimensional space. More specifically, the user terminal 2 measures a coordinate system from the positions of the markers 103, 113, 123 on the head mount apparatus 1 worn on a front face region of the head on the basis of a layout of the characteristic regions instanced by the eyes, the nose and the mouth. The user terminal 2 may also measure the distance by using not only the positions of the markers 103, 113, 123 but also marker area sizes, a width (a height in a vertical direction) of the base member 100, and positional information of the apertures 114, 124 and the scales (termed also gauge portions) of the apertures 114, 124. For example, let S1, S2 be measurement target area sizes in given positional measurements L1, L2, and a distance may be obtained from a relationship:

$$L2/L1 = (S1)^{1/2} / (S2)^{1/2}$$

For example, when values of L1, S1, S2 are known, the user terminal 2 can obtains a distance L2 according to an equation of the inverse proportion given above. It may also be sufficient that the distances to the target markers 103, 113, 123 from the image capturing unit 28 are computed based on the area sizes of the markers 103, 113, 123.

It may be sufficient that the user terminal 2 specify coordinates of three-dimensional positions of the respective regions of the user's head, based on a horizontal line connecting the right and left eyes, the central line of the face, a breadth of the face and a vertical length of the head of the user. Note that the coordinates of the three-dimensional positions of the respective regions may also be specified by previously generating a plurality of three-dimensional models assuming the human head, and selecting the three-dimensional model suited to dimensions of the horizontal line connecting the right and left eyes, the central line of the face, a breadth of the face and the vertical length of the head of the user.

The three-dimensional coordinates of the respective portions of the head mount apparatus 1 and the three-dimensional coordinates of the user's head become coordinates defined in the same three-dimensional space by making origins coincident with each other. It may be sufficient that, e.g., a middle point of the line segment connecting centers of the right and left eyes of the user is set as the origin of the three-dimensional space. On the other hand, the three-dimensional coordinates of the respective portions of the head mount apparatus 1 may be obtained based on, e.g., the central marker 103. It may be sufficient that the coordinate system of the user's head is made coincident with the coordinate system of the head mount apparatus 1 by shifting a relative distance between the position of the central marker 103 and the middle point o the line segment connecting the centers of the right and left eyes of the user.

Thus, the target positions of the user's head and the present positions of the sensors 115, 125 are computed in the three-dimensional coordinate system having the origin determined by reference points, e.g., the eyes, the nose, the mouth and the outline of the head of the user. The target positions and the present positions of the sensors 115, 125 in the three-dimensional coordinate system are converted into the positions in a two-dimensional coordinate system, whereby the positions are displayed in superposition on the image of the user's head.

Through the procedure described above, the user terminal 2 guides the user so that the present positions of the sensors 115, 125 of the head mount apparatus 1 are made coincident with the brain bloodflow measurement position guide frame by being displayed in superposition on the image of the head. It does not, however, mean that the calibration is limited to these processes. For example, the user terminal 2 may guide the user so that the characteristic points, e.g., the upper and lower edges of the base member 100, the markers 103, 113, 123 and the knobs 112, 122 of the head mount apparatus 1 are located in the target positions of the image of the user's head. In other words, it may be sufficient that the positions of the characteristic points of the head mount apparatus 1 are prescribed as the target positions per brain application on the image (the two-dimensional coordinates) of the user's head. In this case also, it may be sufficient that the user is previously given such an instruction that the relative positions of the knobs 112, 122 within the apertures 114, 124 become the predetermined knob positions (default positions) per brain application to be run by the user. It may be sufficient that the user makes an adjustment by moving the characteristic points of the head mount apparatus 1 to the target positions displayed by the user terminal 2 so as not to move the relative positions of the knobs 112, 122 within the apertures 114, 124.

(2) Calibration from Second Time onward

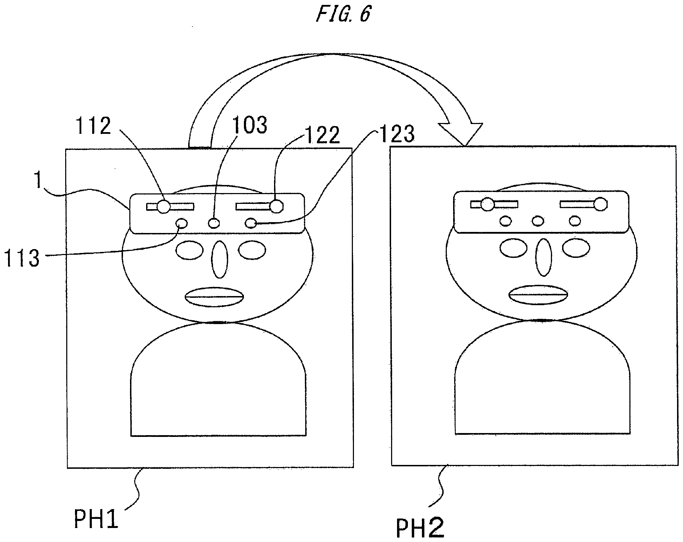

FIG. 6 illustrates a process of the calibration of the second time onward. When the user reuses the already-used brain application, there is used the image of the user's head, which is obtained when the calibration is conducted in the past. In the measurement system, for example, the image (which is referred to as a reference image) of the user' head wearing the head mount apparatus 1 in the desirable layout position acquired in the calibration of the first time, is saved in the memory 22 (the non-volatile storage device) of the user terminal 2. This being the case, the user terminal 2 disposes the reference image (PH1) on the display unit 25, and displays the present head image (PH2) of the user in superposition. For example, the user, at first, adjusts a dimension of the reference image (PH1) to a dimension of the present head image (PH2) by controlling the distance to the image capturing unit 28 of the user terminal 2. The position and the posture of the user's head are modified so that the head in the reference image (PH1) is coincident with the head in the head image (PH2). For instance, it may be sufficient that the posture of the user himself or herself is modified so as to superpose the eyes, the nose and the outline in the present head image (PH2) on the reference image (PH1). The user may also simply modify the wearing state of the head mount apparatus 1 so that the layout of the head mount apparatus 1 in the present head image (PH2) is coincident with the reference image (PH1). For example, it may be sufficient that the adjustment is made to superpose the markers 103, 113, 123 and the knobs 112, 122 of the present head image (PH2) on the respective portions of the reference image (PH1).

The user terminal 2 runs, e.g., an alignment application for supporting the user to align the sensors 115, 125 as described above. After running the alignment application, the user terminal 2 runs the brain application, thereby providing various items of information to the user or the service provider that provides the services to the user, based on the variation of the bloodflow rate at the brain measurement target region of the user.

<Examples of Services and Functions to Be Provided>

The services or the functions provided by the measurement system described above can be exemplified as follows. In the measurement system, the user terminal 2 runs the brain application, whereby the user terminal 2 as a single equipment may provide the services or the functions to the user. The program, i.e., the brain application or the browser and other equivalent programs may access the carrier server 3 via the network N2, and the carrier server 3 may provide the serves or the functions to the user terminal 2.

(a) Providing the User with Information Pertaining to the Brain Activity States of the User: For example, the user terminal 2 or the carrier server 3 (which will hereinafter be generically termed the user terminals 2) can present, to the user, the information indicating the brain activity states in the form of a graph, a table and other equivalent formats.

(b) Display of Image : The user terminals 2 can present, to the user, the information indicating the brain activity states as various types of images. The image contains color variations and brightness (luminance) variations.

(c) Providing the User with Physical Effects Containing at least One of Sounds, Voices, Vibrations and Light: The user terminals 2 may provide, to the user, physical effects containing at least one of sounds, voices, vibrations and light on the basis of the measured activity states of the brain. Herein, the physical effects are exemplified by providing music and musical compositions suited to the brain activity states of the user, controlling the vibrations of the massage machine and controlling an interior illumination.

(d) Providing Information to Participants (in the Schools, Cramming Schools, Sports Clubs) in Present Activities of User: The user terminals 2 may provide the information pertaining to the brain activity states of the user in the form of the graph, the table and the image to participants participating in the present activities of the user as instanced by lecturers, teachers, instructors and coaches of schools, cramming schools and sports clubs. The schools, the cramming schools and the sports clubs are thereby enabled to give instructions suited to the brain activity states of the user.

(e) Controlling Apparatus (Personal Computer, Tablet Computer, on-Vehicle Device of Car, Installed with Learning Application for Children) or Facilities (Instanced by Schools, Cramming Schools, Sports Clubs) Currently in Active Use by User: For instance, based on a case that the brain of the user is in an inactive state, the user may be provided with a stimulus of activating the brain of the user from the apparatus instanced by the personal computer and the on-vehicle device of the car, which are installed with the learning application, or the facilities instanced by the schools, the cramming schools and the sports clubs. The stimulus described above is a stimulus instanced by displaying on the display, the voice, the sound, the physical vibration and the light. For example, the on-vehicle device may give a physical stimulus (a display-based visual stimulus, and a voice/sound-based auditory stimulus) for preventing drowsiness to a driver when determining that the user's brain is in the inactive state from the measurement data of the bloodflow variations of the head mount apparatus 1. The on-vehicle device determines the measurement data of the bloodflow variations, and may guide the driver to take a rest. The computer of the facility instanced by the school, the cramming school and the sports club, when determining that the user's brain is in the inactive state from the measurement data of the bloodflow variations of the head mount apparatus 1, may give the individual participants such a stimulus as to be being watched by displaying the information for identifying the participants with the brains becoming inactive or displaying the brain states of the individual participants. The computer may acquire the data of the variations of the bloodflow rate measured by the head mount apparatus 1 via the user terminal 2, and may also acquire the data of the variations of the bloodflow rate directly from the head mount apparatus 1 via the network N1 illustrated in FIG. 1.

(f) Transmission of Information to Apparatuses (other smartphones, PCs) Cooperating with Apparatus Currently in Active Use by User: The user terminal 2 may transmit the brain activity states of the user to other smartphones, the PCs or facilities instanced by a TV broadcasting station. For example, the computer of the TV broadcasting station acquires measurement values indicating brain activity states of viewers by being associated with programs and advertisements to be broadcasted, and is thereby enabled to output the brain activities exhibiting sensitivities of the viewers to the programs and reactions to the advertisements on the basis of the variations of the bloodflow rate of the brain functional region. In this case, the advertisements may be given by dynamic images (videos) and may also be given by static image (still photos).

(g) Information Pertaining to Brain Activity States of User: The user terminal 2 or the carrier server 3 provides the users or the service providers for providing the services to the user in a variety of formats or modes with the information about the brain activity states of the user on the basis of the measurement data of the variations of the bloodflow rate that are measured by the head mount apparatus 1. For instance, the user terminal 2 or the carrier server 3 may also provide information representing a present state by comparing evaluation values accumulated in the past with respect to the brain activity states of the user with a present evaluation value. The user terminal 2 or the carrier server 3 may also provide information representing correlations between the evaluation values of the brain activity states of the user and other items of human information of the user. The user terminal 2 or the carrier server 3 may further provide information correlations between the evaluation values of the brain activity states of the user and physical conditions of the user. The user terminal 2 or the carrier server 3 may still further provide information correlations between the evaluation values of the brain activity states of the user and mental conditions of the user. The user terminal 2 or the carrier server 3 may yet further provide information correlations between the evaluation values of the brain activity states of the user and information provided on the Internet.

(h) Application to Feedback Training: Athletes emphasize self-control and therefore excise feedback training of heart beats. For example, the feedback training using brain waves is carried out in U.S.A. The user wears the head mount apparatus 1 and runs the brain application on the user terminal 2, thereby enabling the user to perform the feedback training by using the measurement data of the variations of the bloodflow rate of the user himself or herself.

[Example 1]

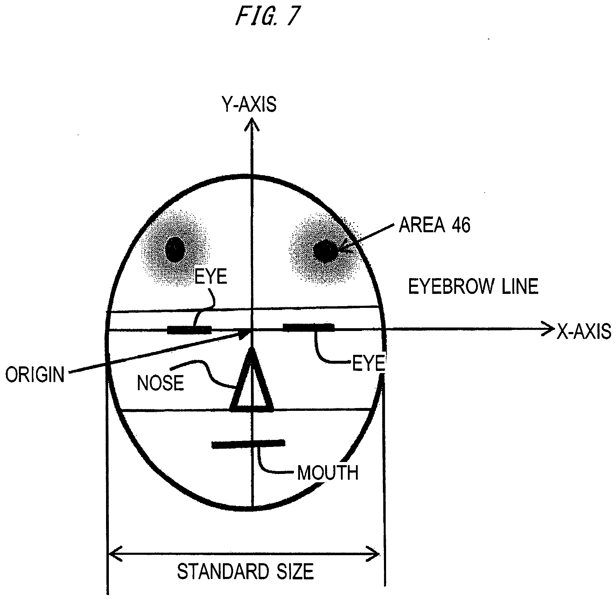

The measurement system according to an Example 1 will hereinafter be described with reference to FIGS. 7 through 17. The system architecture of the measurement system according to the Example 1 and the structure of the head mount apparatus 1 are the same as those depicted in FIGS. 2-4. The Example 1 will describe a processing example that the user terminal 2 performs the calibration per brain application, based on the image of the user's head, which is captured by the image capturing unit 28.

<Three-Dimensional Model of Standard Size>

The Example 1 will describe a processing example of the user terminal 2 based on the alignment application for guiding the user to align the sensors 115, 125. FIG. 7 is a view illustrating a model coordinate system of the user in the Example 1. The user terminal 2 in the Example 1 sets an X-axis on the straight line connecting the right and left eyes on the image of the user's head that is captured by the image capturing unit 28. As for the direction, the right direction toward the user is defined as a forward direction. The user terminal 2 sets a Y-axis with an upward direction being defined as the forward direction on the vertical straight line passing through the center of the user's nose. Accordingly, the X-axis and the Y-axis intersects at a middle point of the line segment connecting the centers of the right and left eyes, and the intersection therebetween becomes an origin. At the origin, the user terminal 2 sets a Z-axis by defining the upward direction viewed vertically from the sheet surface in FIG. 7 as the forward direction. The user terminal 2 sets the three-dimensional coordinate system of the human head in the manner described above.

The user terminal 2 has such a model that an actual size of the user's head is changed to a standard size. The standard size is prescribed by a breadth of the face, and may involve using a typical size of a person. The user terminal 2 has data, in the three-dimensional coordinate system, of layout positions of the sensors 115, 125 associated with each brain application on the model of the standard size. Therefore, the user terminal 2 converts the head image of the user's head, which is captured by the image capturing unit 28, into the model of the standard size, and further converts a wearing position of the headmount apparatus 1 in the two-dimensional coordinate system on the head image into a wearing position in the three-dimensional coordinate system of the model of the standard size. The user terminal 2 displays objects serving as guides on the head image displayed in the two-dimensional coordinate system on the display unit 25 so that the sensors 115, 125 are disposed in target positions per brain application in the model of the standard size, thus supporting the user to align the sensors 115, 125.

<Data Structure>

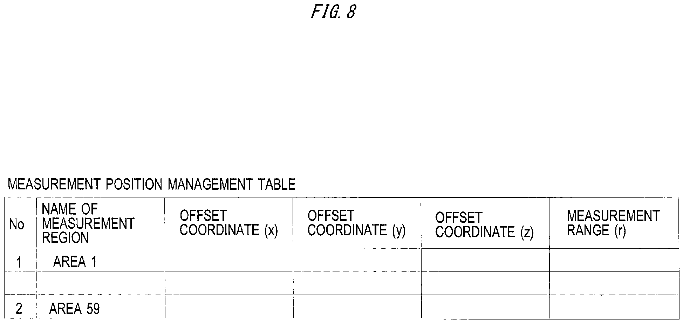

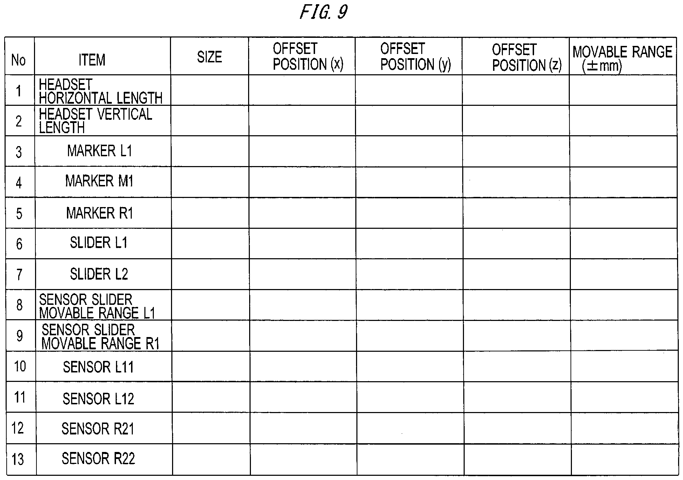

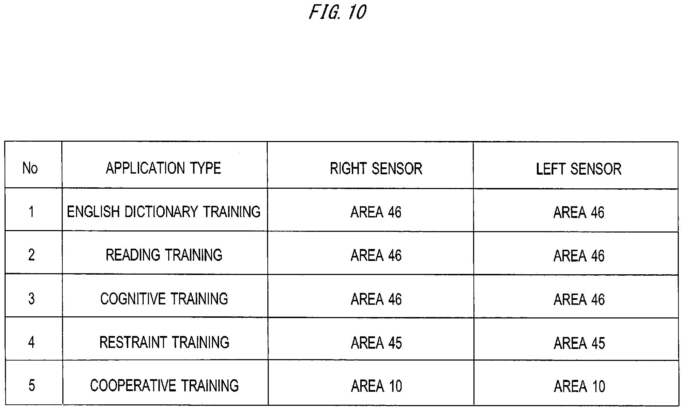

FIG. 8 is a diagram illustrating an example of a measurement position management table that defines a relationship between the measurement target region in a three-dimensional model of the standard size and the target positions of disposing the sensors 115, 125 in the model. In the Example 1, the human brain is segmented into a plurality of regions, and the target positions of disposing the sensors 115, 125 are defined in order to measure the variations of the bloodflow rate of each of the segmented regions. The measurement position management table in FIG. 8 is retained in the memory 22 of the user terminal 2. In the table of FIG. 8, however, a first row is a descriptive row, and rows from a second row onward are actually retained in the memory 22. The point that the first row of the table does not contain the data to be retained in the memory 22 but is the descriptive row, is likewise applied to FIGS. 9 and 10.

Each row of the measurement position management table in FIG. 8 has respective fields, i.e., a "No" field, a "measurement region name" field, an "offset coordinate (x)" field, an "offset coordinate (y)" field, an "offset coordinate (z)" field, and a "measurement range (r)" field. A value of the "No" field is information for identifying the row (record) in the measurement position management table. However, the "No" field may be omitted in the measurement position management table. This is because the respective rows (records) in the measurement position management table can be identified by the measurement region names.

The "measurement region name" field contains information specifying the measurement target region of the human brain. A variety of segmenting methods are proposed for segmenting the human brain. For example, one method is that the brain is segmented based on a structure of the brain into a cerebra, an interbrain, a cerebella, and a brainstem: and further, the cerebra is segmented into a frontal lobe, a lobus parietalis, an occipital lobe and a lobus temporalis. For example, Korbinian Brodmann proposed a brain map structured such that regions each having a uniform tissue structure in a brain cortex are classified on a clump-by-clump basis as areas to which "1" through "52" are allocated. In the Example 1, similarly to the Brodmann's brain map, numerals are allocated to the respective regions (areas) of the brain cortex, and are adopted as the measurement region names. In FIG. 8, the measurement region names are exemplified by areas 1 - 59. In other words, when the brain cortex is segmented to make the measurement in the present measurement system, it does not mean that the segmentation is limited to the same segmentation (classification) as the Brodmann's brain map. It may be sufficient that the present measurement system measures the variations of the bloodflow rates of the respective segmented regions in the way of being suited to an intended usage or an application of the measurement data by using a standard brain map equivalent to the Brodmann's brain map, or a brain map uniquely defined by a maker, or a brain segmentation in the standard brain coordinates.

For example, when the measurement position management table is defined by use of the Brodmann's brain map and when limited to the frontal lobe, the table in FIG. 8 may be structured to have six rows (records) containing an area 9, an area 10, an area 11, an area 45, an area 46 and an area 47.

The offset coordinate (x), the offset coordinate (y) and the offset coordinate (z) are the target positions of disposing the sensors 115, 125 in order to measure the respective regions identified by the measurement region names, and are also coordinate values in the three-dimensional coordinate system. Herein, the three-dimensional coordinate system is the coordinate system illustrated in FIG. 7. The positions of the respective regions are the positions in the model of the standard size illustrated in FIG. 7. The measurement range (r) covers allowable errors from the target positions described above, e.g., radii from the offset coordinate (x), the offset coordinate (y) and the offset coordinate (z).

Thus, the user terminal 2, when determining the measurement target position per brain application owing to retaining the measurement position management table, disposes the sensors 115, 125 in the target positions of the model of the standard size in the three-dimensional coordinate system. The user terminal 2 obtains the target positions of disposing the sensors 115, 125 by converting the positions in the three-dimensional coordinate system into those in the two-dimensional coordinate system. It may be sufficient that the user terminal 2 displays the multiple guides on the head image of the user on the basis of the obtained target positions in the two-dimensional coordinate system, and supports the user for disposing the sensors 115, 125.

FIG. 9 illustrates an example of a structure management table of the head mount apparatus 1 (illustrated as a headset in FIG. 9). The structure management table defines dimensions or positions of the respective units of the head mount apparatus 1. In the Example 1, however, the dimensions and the positions are given not in a state of the head mount apparatus 1 being bent but in a state of the head mount apparatus 1 being spread on the plane. FIG. 9 illustrates a headset horizontal length, a headset vertical length, markers L1, M1, R1, sliders L1, L2, sensor slider movable ranges L1, R1, and positions of sensors L11, L12, R11, R12. It does not, however, mean that the structure management table is limited to the definitions of the respective units in FIG. 9.

Note that the markers L1, M1, R1 correspond to the markers 103, 113, 123. The sliders L1, L2 correspond to the knobs 112, 122. For example, the sensors L11, L12 correspond to the two light receiving units of the sensor 115, and the sensors R11, R12 correspond to the two light receiving units of the sensor 125. Note that totally the four sensors are defined in FIG. 9, but it does not mean that the number of the sensors is limited to "4" in the Example 1.

Each of rows (records) in FIG. 9 has respective fields (elements), i.e., a "size" field, an "offset coordinate (x)" field, an "offset coordinate (y)" field, an "offset coordinate (z)" field and a "movable range" field. Of these fields, the "size" field retains dimensions of the respective units. The "offset coordinate (x)" field, the "offset coordinate (y)" field and the "offset coordinate (z)" field retain coordinates of positions in which reference points of the respective units of the head mount apparatus 1 are disposed. The offset coordinates are coordinates of the coordinate system within the head mount apparatus 1, in which, e. g., the marker M1 is set as the origin. The coordinate system in the head mount apparatus 1 is defined by the X-axis, passing through the markers L1, M1, R1, along which the forward direction is the right direction toward the user wearing the head mount apparatus 1 and by the Y-axis (the coordinate system on the right side) passing through the marker M1 (origin) and vertical to the X-axis. However, the Example 1 defines such a shape that the head mount apparatus 1 is spread on the plane. Hence, a value of the offset coordinate (z) is normally "0". The movable range is an element (value) that is set in each of the sensor slider movable ranges L1, R1.

The user terminal 2 includes the structure management table in the memory 22 and is thereby enabled to recognize a degree of bending of the head mount apparatus 1 and a distance to the image capturing unit 28 from the head mount apparatus 1 on the basis of the shape (e.g., a ratio of the horizontal length to the vertical length of the headset) of the head mount apparatus 1, the layout of the components (the positions of the markers 113, 123 with respect to the marker 103) and the area sizes of the components, which are obtained on the image of the user's head. To be specific, the user terminal 2 computes the positions of the sensors 115, 125 in the three-dimensional coordinate system on the basis of the sizes, the offset positions and other equivalent values of the structure management table, and the area sizes and positions of the respective units on the image. The user terminal 2 obtains, as illustrated in FIG. 7, the present positions of the sensors 115, 125 with respect to the origin on the head image acquired from the characteristic points (eyes, nose and other equivalent regions) of the image of the user's head. The user terminal 2 converts the present positions obtained from the image in the three-dimensional coordinate system into the positions of the model of the standard size. Accordingly, the user terminal 2 can obtain the present positions of the sensors 115, 125 in the model of the standard size. The user terminal 2 obtains the target positions of the sensors 115, 125 in the model of the standard size. The user terminal 2 converts the present positions and the target positions of the sensors 115, 125 into the positions on the image in the two-dimensional coordinate system, which is captured by the image capturing unit 28. The user terminal 2 is thus enabled to guide the user by displaying the present positions of the sensors 115, 125 and the target positions in which to dispose the sensors 115, 125 on the image of the user' s head, which is captured by the image capturing unit 28.

FIG. 10 is a diagram of an example of a sensor slider set value management table. The sensor slider set value management table prescribes the measurement regions per type (training) of the application (brain application). As already explained in FIG. 8, the positions of disposing the sensors for measuring the respective measurement regions are set in the measurement position management table. Upon determining the brain application to be run, the user terminal 2 therefore reads the measurement region assigned to each sensor by searching the relevant brain application from the sensor slider set value management table. The user terminal 2 reads the positions of disposing the sensors for measuring the respective measurement regions from the measurement position management table, and displays the objects (which are also called the guides) serving as the target positions used for disposing the sensors in the relevant positions on the image of the user's head.

Each of rows (records) of the sensor slider set value management table has respective fields (elements), i.e., an "application type" field, a "right sensor" field and a "left sensor" field.

A name of the brain application to be run by the user terminal 2 or identifying information or an identification code of the brain application is designated in the "application type" field. The example of FIG. 10 is that English dictionary training, oral reading training, recognition training, restraint training and collaborative training are exemplified as the types of the brain application. The collaborative training is the training for enforcing, e.g., a teamwork. The "right sensor" and the "left sensor" are information for identifying the respective sensors, and the measurement target regions assigned to the respective sensors are designated in the elements ("right and left sensor" fields) of each corresponding row. As already explained in the measurement position management table in FIG. 8, it may be sufficient that the brain regions are defined by using the standard brain map equivalent to the Brodmann's brain map, or the brain map uniquely defined by the maker, or the brain segmentation in the standard brain coordinates.

Note that the head mount apparatus 1 includes the knobs 112, 122, the sensor 115 is slidable by the knob 112, and the sensor 215 is slidable by the knob 122 as illustrated in FIGS. 2 through 4. The sensor 115 corresponds to the left sensor, while the sensor 125 corresponds to the right sensor. Accordingly, the left sensor is movable by the knob 112, while the right sensor is movable by the knob 122.

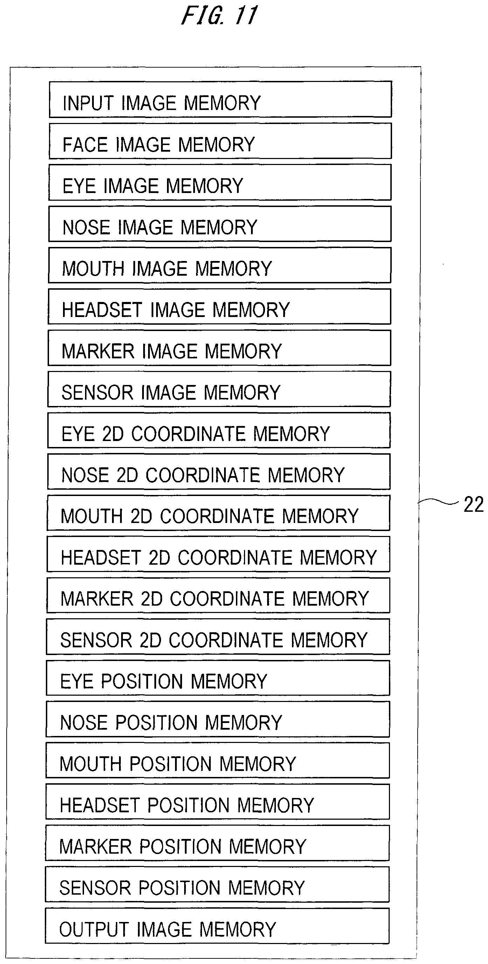

FIG. 11 is a diagram of an example of data retained in the memory 22 by the user terminal 2. In FIG. 11, the respective items of data are specified by area names in the memory 22. For example, an input image memory of the memory 22 connotes a buffer area to retain the data of the image captured by the image capturing unit 28. Image frames are written at a predetermined frame interval (cycle) to the input image memory defined as the buffer area from the image capturing unit 28.

A face image memory retains image data of a facial region of the user, which is recognized by the user terminal 2 (alignment application), in the image data, written to the input image memory, of the user's head. An eye image memory, a nose image memory and a mouth image memory retain image data of the respective regions, i.e., the eyes, the nose and the mouth of the user, which are recognized by the user terminal 2 (alignment application), in the image data, written to the input image memory, of the user's head. A headset image memory and a marker image memory retain image data of the head mount apparatus 1 and image data of the markers, which are recognized by the user terminal 2 (alignment application), in the image data, written to the input image memory, of the user's head. A sensor image memory retains image data of marks indicating the target positions of disposing the sensors.

An eye 2D coordinate memory retains coordinate values of the two-dimensional coordinate system, which indicate positions of the eyes in the head image recognized by the user terminal 2. Note that the middle point of the line segment connecting the centers of the right and left eyes of the user is, as already described, set as the origin in the two-dimensional coordinate system (see the X- and Y-axes in FIG. 7).

The same is applied to a nose 2D coordinate memory and a mouth 2D coordinate memory. A headset 2 D coordinate memory retains a coordinate value indicating a position of a reference point of the present head mount apparatus 1 in the two-dimensional coordinate system. A maker 2D coordinate memory and a sensor 2D coordinate memory respectively retain coordinate values indicating a position of the maker and a position of the sensor in the two-dimensional coordinate system. An eye position memory, a nose position memory, a mouth position memory, a headset position memory, a marker position memory and a sensor position memory retains coordinate values indicating positions of the eyes, the nose, the mouth, the reference point of the head mount apparatus 1, the marker and the sensor in the three-dimensional coordinate system.

<Example of Alignment Process>

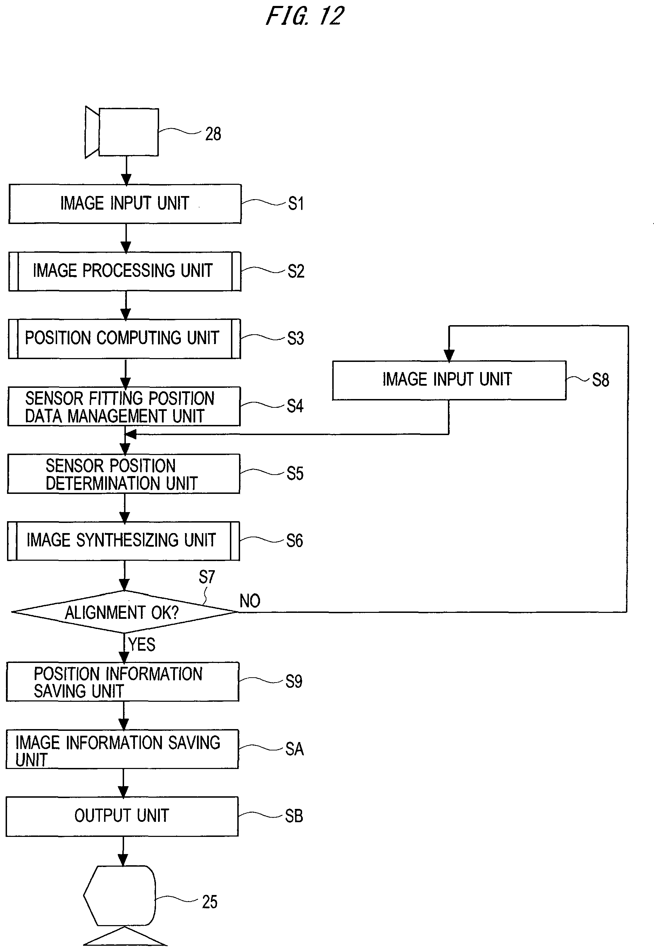

FIG. 12 is a flowchart illustrating an alignment processing procedure of the first time. The CPU 21 executes processes in FIG. 12 in accordance with an alignment application as a computer program on the memory 22. When the user instructs the user terminal 2 to run the desired brain application, the CPU 21 executes the processes in FIG. 12 before running the brain application.

In this process, at first, the CPU 21 acting as an image input unit executes a process, thereby acquiring the image captured by the image capturing unit 28 (S1). Subsequently, the CPU 21 acting as an image processing unit executes a process (S2). In the process of S2, the CPU 21 recognizes the characteristic points of the image of the user's head from, e.g., the acquired image in S1. Next, the CPU 21 acting as a position computing unit executes a process (S3). In the process of S3, the CPU 21 computes coordinate values of the characteristic points recognized in S2.

Subsequently, the CPU 21 acting as a sensor fitting position data management unit executes a process (S4). In the process of S4, the CPU 21 acquires the sizes of the respective units of the head mount apparatus 1, the offset positions (x, y, z), the movable range and other equivalent items from the structure management table of the head mount apparatus 1 as illustrated in FIG. 10.

The CPU 21 acting as a sensor position determination unit executes a process (S5). In the process of S5, the CPU 21 computes the present positions (three-dimensional coordinate system) of the sensors from the sizes of the respective units of the head mount apparatus 1, the offset positions (x, y, z) and the positions of the characteristic points in S2. The CPU 21 also acquires the measurement regions by the respective sensors on a size-by-size basis of the user' head per brain application from the sensor slider set value management table illustrated in FIG. 10. The CPU 21 also acquires the sensor offset positions corresponding to the measurement regions by the sensors from the measurement position management table illustrated in FIG. 8. The offset positions of the sensors are disposed on the model with the size of the user's head being converted into the standard size. These offset positions are the target positions of disposing the sensors, corresponding to the brain application. The CPU 21 converts a model image, in which the present positions and the target positions of the sensors are disposed, into a two-dimensional image. The two-dimensional image is of the two-dimensional coordinate system in which the middle point of the line segment connecting the centers of the right and left eyes of the user is the origin. The CPU 21, when converting the model image into the two-dimensional image, converts the head size of the model into a head size of the user from the standard size, thereby obtaining the present positions and the target positions of the sensors in the two-dimensional coordinate system. The CPU 21 renders the objects serving as the guides in the present positions and the target positions of the sensors in the two-dimensional coordinate system.

Next, the CPU 21 acting as an image synthesizing unit executes a process (S6). In the process of S6, the CPU 21 superposes the two-dimensional image generated by disposing the sensors in the model in the process of S5 on the image of the user's head, which is acquired from the image capturing unit 28.

Subsequently, the CPU 21 determines whether the alignment is sufficiently conducted (S7) . A case of the sufficient alignment being conducted connotes, e.g., a case that the positions of the sensors fall within an allowable error range of the measurement region, which is prescribed per application in the model of the standard size. When the determination is negative in S7, the CPU 21 prompts the user to modify the present positions of the sensors so as to get close to the target positions, and executes a process in S8 as an image input unit and the process in S5 as the sensor position determination unit. Specifically, the CPU 21 acquires again the image of the user's head from the image capturing unit 28, and renders the objects serving as the guides in the present positions and the target positions of the sensors in the two-dimensional coordinate system. The CPU 21 executing the processes in S1 through S8 is one example of "means to support an adjustment by locating means".

Whereas when determining in S7 that the alignment is sufficiently conducted, the CPU 21 acting as a positional information saving unit executes a process (S9). In the process of S9, the CPU 21 saves, e.g., the present positions of the eyes, the nose and other equivalent regions, and the positions of the markers 103, 113, 123 in the memory 22.

The CPU 21 acting as an image information saving unit executes a process (SA). In the process of SA, the CPU 21 saves the two-dimensional image synthesized in S6 as a reference image in the memory 22. The CPU 21 executes a process as an output unit, and outputs information indicating a success in the alignment. For example, the CPU 21 executes any one or a combination of outputting a message to the display unit 25 and outputting the sounds or the vibrations to the output unit 27 (SB).

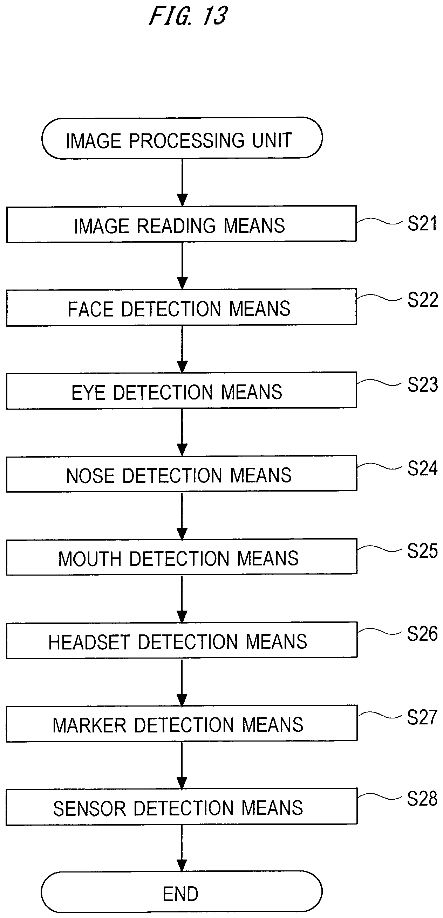

FIG. 13 is a flowchart illustrating details of the process (S2 in FIG. 12) of the image processing unit. In this process, the CPU 21 acting as image reading means executes a process (S21). For instance, the CPU 21 acquires the image retained in the frame buffer and other equivalent storages. The CPU 21 stores the acquired image in, e.g., an image memory within the memory 22.

Next, the CPU 21 acting as face detection means executes a process (S22). In this process, the CPU 21 identifies a face region from the image. The CPU 21 stores the acquired image of the face region in, e.g., a face image memory within the memory 22. Subsequently, the CPU 21 acting as eye detection means executes a process (S23). In this process, the CPU 21 identifies eye regions from the image. The CPU 21 stores the acquired images of the eye regions in, e.g., the image memory within the memory 22. Next, the CPU 21 acting as nose detection means executes a process (S24). In this process, the CPU 21 identifies a nose region from the image. The CPU 21 stores the acquired image of the nose region in, e.g., the image memory within the memory 22. Subsequently, the CPU 21 acting as mouth detection means executes a process (S25). In this process, the CPU 21 identifies the mouth region from the image. The CPU 21 stores the acquired image of the mouth region in, e.g., the image memory within the memory 22. The processes in S22 through S25 are the same as those of detecting the face, the eyes, the nose and the mouth in a face recognition process executed by a general type of digital camera and other equivalent imaging devices.

Subsequently, the CPU 21 acting as headset detection means executes a process (S26). In the process of S26, it may be sufficient that the CPU 21 performs template matching with the image acquired in S21 by using, e.g., a template image of the head mount apparatus 1 (headset). The CPU 21 stores the acquired image of the head mount apparatus 1 in, e.g., a headset image memory within the memory 22. Next, the CPU 21 acting as marker detection means executes a process (S27). The process in S27 is the same as the process in S26. The CPU 21 stores the acquired image containing the markers 103, 113, 123 in, e.g., a marker image memory within the memory 22.

Further, the CPU 21 acting as sensor detection means executes a process (S28). In the process of S28, the CPU 21 computes the present positions of the sensors 115, 125 on the rear face side of the head mount apparatus 1 from the positions and the dimensions of the markers 103, 113, 123 of the head mount apparatus 1, an intra-image dimension of the base member 100 and the positions of the knobs 112, 122. As already explained, the present positions of the sensors 115, 125 are obtained in the three-dimensional coordinate system by being converted into the positions in, e.g., the standard model. In the process of S28, however, the present positions of the sensors 115, 125 are obtained in the coordinate system (with the origin being the marker M1 (marker 103)) of the head mount apparatus 1. This is because the positions of the characteristic points of the face, the eyes, the nose and other equivalent regions of the user are not yet obtained.

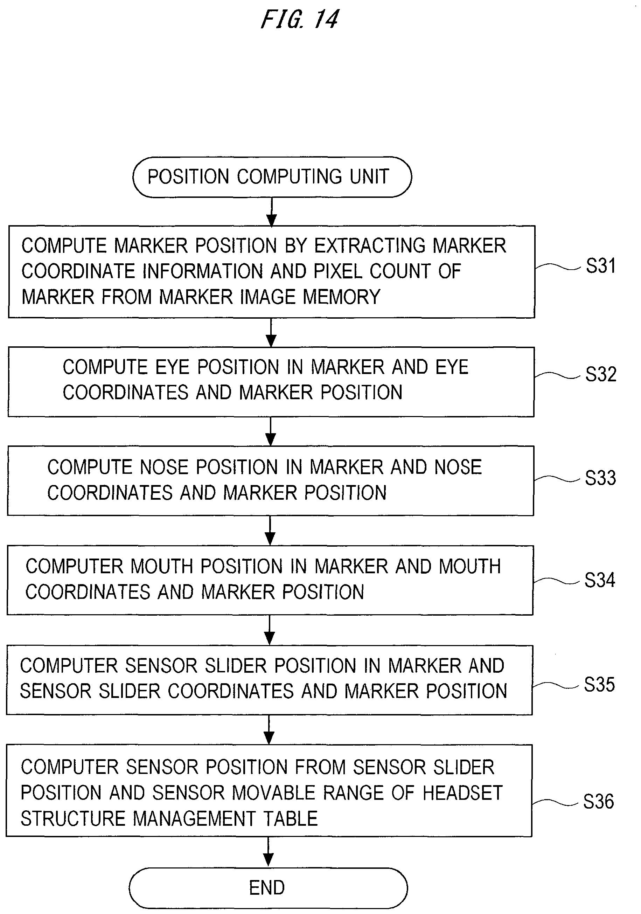

FIG. 14 is a flowchart illustrating details of the process (S3 in FIG. 12) of the position computing unit. In this process, the CPU 21 extracts a number of pixels (pixel count) of the marker from marker coordinate information and the marker image memory, and computes actual positions of the markers in the three-dimensional coordinate system from the two-dimensional coordinate system on the image by obtaining a physical size (mm/pixel) corresponding to the pixels of the marker image 1 on the head mount apparatus 1 and a curvature of the head mount apparatus 1 on the basis of physical sizes of the markers and information of layout correlation of the markers in a headset structure diagram (drawing within an electronic file defining the physical sizes and the layout relation, or a group of parameters defining the dimensions of the respective units, or both of the drawing and the group of parameters) defining the structure of the headmount apparatus 1 (S31). More specifically, the memory 22 retains, as the information, the sizes and the layout in the headset structure diagram, and hence the CPU 21 computes relative distances by comparing, e.g., the sizes (especially the size of the marker 103) of the markers 103, 113, 123 acquired from the marker images within the memory 22 with the known sizes of the three markers (especially the size of the central marker) of the head mount apparatus 1. For example, the CPU 21 can compute a structural distortion of the head mount apparatus 1, specifically a curvature of how much the head mount apparatus 1 is distorted along the (outline of) human face on the basis of a comparison between the known layout information of the markers provided right and left in the headset structure diagram and the information of the marker images (especially the markers 113, 123 provided on the right and left sides of the head mount apparatus 1) within the memory 22. The CPU 21 can compute the actual positions of the markers 103, 113, 123 in the three-dimensional coordinate system from these items of information. Through the same procedure, the CPU 21 computes the eye positions with respect to the marker coordinates and the eye coordinates in the two-dimensional coordinate system of the image and the marker positions in the three-dimensional coordinate system (S32). Herein, the eye positions are located under the position of the central marker 103 in the structure of the head mount apparatus 1. Note that a central position between the eyes and a distance between the two eyes can be obtained corresponding to the position of the general head mount apparatus 1 by making use of statistic data. This being the case, the CPU 21 may compute the eye positions from the marker coordinates by using the memory 22 containing the statistic data of a distance between the position (the positions of the markers 103, 113, 123) of the head mount apparatus 1, an eye height (a Y-axis directional position of the line segment connecting the centers of the right and left eyes) and a distance between the two eyes (a length of the line segment connecting the centers of the right and left eyes, a length between eye corners or a length between inner corners of the eyes). Note that the CPU 21 may also specify the eye positions within the image through pattern matching by using a database of eye image patterns, which is provided in the memory 22, in place of employing the statistic data described above. In this case, e.g., the actual eye positions may also be specified from a corresponding relation between the eye positions within the image and the positions of the markers, a corresponding relation between the positions of the markers 103, 113, 123 within the image and the actual positions of the markers 103, 113, 123 in the three-dimensional coordinate system, and the curvature drawn out by the above. These corresponding relations may be retained in a table of the database retained on the memory 22. The memory 22 retains parameters in an experimental formula in these corresponding relations, and the CPU 21 may be thereby enabled to compute a function relation.

Next, the CPU 21 computes the nose position with respect to the marker coordinates and the nose coordinate in the two-dimensional coordinate system of the image and the marker positions in the three-dimensional coordinate system (S33). The nose position can be also specified by using the statistic data or nose pattern matching in the same way as specifying the eye coordinates. Subsequently, the CPU 21 computes mouth position with respect to the marker coordinates and the mouth coordinate in the two-dimensional coordinate system of the image and the marker positions in the three-dimensional coordinate system (S34). The mouth position can be also specified in the same way as specifying the eye positions and the nose position. Subsequently, the CPU 21 computes sensor slider positions with respect to the marker coordinates and the sensor slider coordinates in the two-dimensional coordinate system of the image and the marker positions in the three-dimensional coordinate system (S35). The same as the above is applied to the sensor slider positions. Note that the sensor slider positions are the positions of the knobs 112, 122 illustrated in FIG. 4. Finally, the CPU 21 computes sensor positions from the sensor slider positions and the sensor movable range in the headset structure management table in the same way as the above.

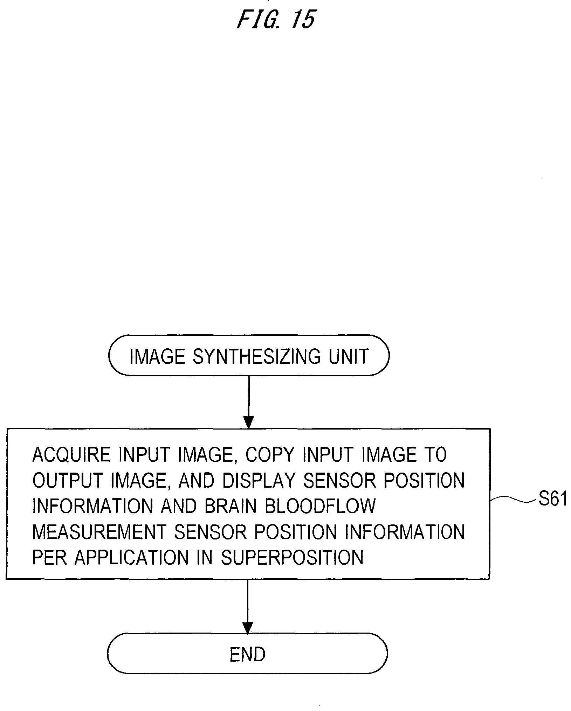

FIG. 15 is a flowchart illustrating details of the process (S6 in FIG. 12) of the image synthesizing unit. In this process, the CPU 21 acquires and maps input images to output images, thus displaying present sensor position information (the objects of the sensors in the present positions) and brain bloodflow measurement sensor position information (objects in the target positions) per application in superposition (S61).

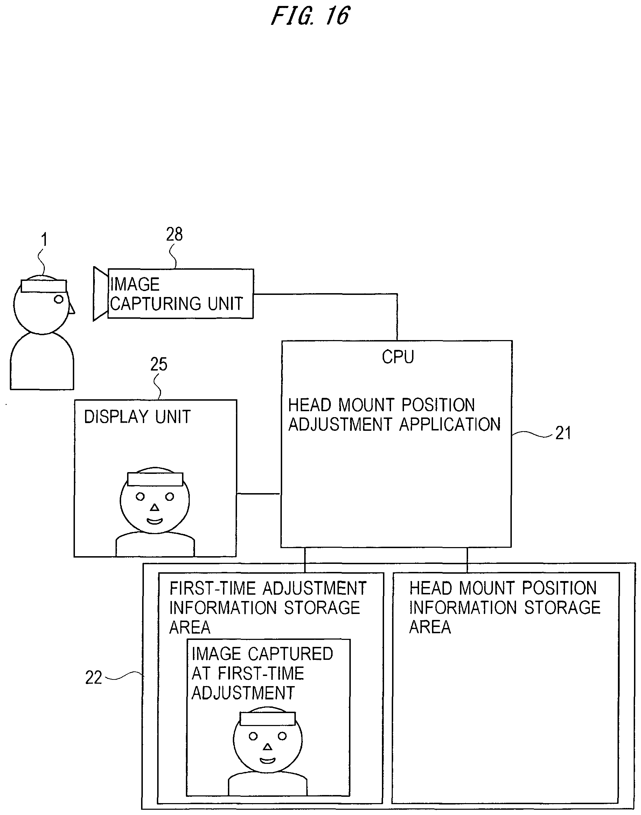

FIG. 16 is a schematic diagram illustrating processes of the calibration of the second time, which is executed when the user uses again the brain application having a result of the actual usage. With respect to the brain application having the result of the actual usage, the sensors are already aligned when running the brain application for the first time. A result of the sensor alignment made based on the calibration when running the brain application for the first time, is saved in a first time adjustment information storage area of the memory 22 as the image captured when making the adjustment of the first time in such a state that the head mount apparatus 1 is mounted on the user's head. The images of the user wearing the head mount apparatus 1 from the second time onward are acquired in a head mount position information storage area, e.g., at the predetermined frame interval (cycle).

Accordingly, when the user uses again the brain application having the result of the actual usage, the user terminal 2 may not re-execute the processes illustrated in FIGS. 12 through 15 as when in the usage of the first time. It may be sufficient that the user terminal 2 guides the user to take, e.g., the same wearing state of the head mount apparatus 1 as the image captured when making the adjustment of the first time in the same distant position from the image capturing unit 28 as when for the first time. The user terminal 2 carries out this guide in accordance with the head mount position adjustment application (alignment application).

In other words, the user terminal 2 displays the present image of the user's head on the display unit 25, and further displays the image captured when making the adjustment of the first time in superposition thereon. It may be sufficient that the user terminal 2 prompts the user to modify the distance from the image capturing unit 28, the posture of the user, the wearing state of the head mount apparatus 1 and the positions of the knobs 112, 122 so that the present image of the user's head becomes the image captured when making the adjustment of the first time.

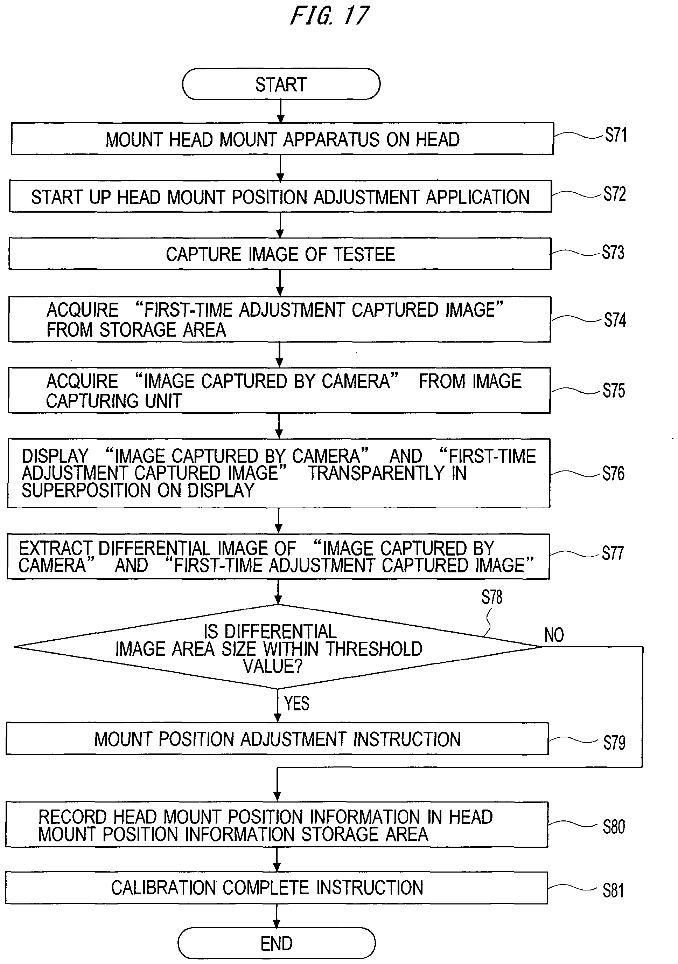

FIG. 17 is a flowchart illustrating processes of the calibration from the second time onward. The user terminal 2, when running the brain application, checks whether the image captured when making the adjustment of the first time corresponding to the brain application is saved, and is thereby enabled to determine whether the calibration from the second time onward can be performed. The user terminal 2, when determining that the calibration is the calibration from the second time onward, starts up processing in FIG. 17. In this process, e.g., the user terminal 2 instructs the user to wear the head mount apparatus 1 (S71). The CPU 21 of the user terminal 2 starts up the headset mount position adjustment application (alignment application) (S72). The following processes are processes of the CPU 21 based on the headmount position adjustment application. in this process, the user directs the image capturing unit 28 of the user terminal 2 toward the user himself or herself. Through this operation, the image capturing unit 28 captures the image of the user's head (S73).

The CPU 21 acquires the image captured when making the adjustment of the first time from the storage area (memory 22) (S74). The image captured when making the adjustment of the first time is one example of a "saved head image". The CPU 21 executing the process in S74 is one example of "means to acquire the saved head image of the user". Next, the CPU 21 acquires, from the frame buffer, the present image (which will hereinafter be termed "camera image"), captured by the image capturing unit 28, of the user himself or herself (S75). The camera image is one example of "the present head image of the user". The CPU 21 executing the process in S75 is one example of "means to acquire a present head image of a user". The CPU 21 displays the camera image and the image captured when making the adjustment of the first time on the display unit 25 (S76). The CPU 21 extracts a differential image between the camera image and the image captured when making the adjustment of the first time (S77). The CPU 21 computes a differential image area size, and determines whether the differential image area size is equal to or smaller than a threshold value (S78). The differential image area size is instanced by an area size of the differential image or a maximum dimension of the image. The CPU 21 integrates the number of pixels within the differential image, and is thereby enabled to compute the area size of the differential image. The CPU 21 may also obtain the maximum dimension of the image by counting the number of pixels of, e.g., a pixel consecutive portion within the differential image. The threshold value can be set as a system parameter of the user terminal 2 from an experimental or an empirical value. When determining in S78 that the differential image area size is not equal to or smaller than the threshold value, the CPU 21 instructs the user to make a mount position adjustment (S79). The instruction in S79 is given in the form of a message to the display unit 25, and a sound, a voice or a vibration from the output unit 27. The CPU 21 loops back the control to S77. Note that the camera image is updated at the predetermined frame interval (cycle) when the control is looped back to S77. The CPU 21 executing the processes in S76 - S79 is one example of "means to support an adjustment of the locating means".

Whereas when determining in S78 that the differential image area size is equal to or smaller than the threshold value, the CPU 21 records the headset mount position information in the headset position information storage area (S80). The CPU 21 instructs each of the units to complete the calibration (S81). The instruction to each of the units is exemplified by an output to the display unit 25 or the output unit 27, or an instruction to run the brain application.

As described above, the measurement system according to the Example 1 enables the user terminal 2 to provide the variety of services to the user, which receives the transfer of the detection data of the detected variations of the bloodflow rate of the user's head. In the measurement system, the user terminal 2 supports the user to perform the alignment by performing the guide using the objects of the present positions and the target positions of the sensors 115, 125 with respect to the coordinate system defined by the dimensions of the markers 103, 113, 123 and the base member 100 and the characteristic points of the eyes, the nose and other equivalent regions of the user. The user is therefore enabled to adjust the posture of the user himself or herself, the position of the head, the distance from the image capturing unit 28 to the user's head and the position of mounting the head mount apparatus 1 on the user's head in accordance with the guide. The user is further enabled to minutely adjust the positions of the sensors 115, 125 to the specified region of the user's head by using the knobs 112, 122.

Particularly when the specified region is the frontal lobe, the measurement system acquires the measurement data pertaining to internal reactions or the reactions about, e.g., languages, actions, perceptions, memories, attentions, determinations, especially, thoughts, creations, intentions and plans of the user, and can provide the user himself or herself with the services corresponding to the internal reactions or states of the user, or can provide these services to business persons and other equivalent persons utilizing the internal reactions or states of the user.

These items of measurement data are transferred via a wireless communication path, in which case the measurement data can be utilized for the variety of services and intended usages. When the alignment support is applied to the proper specified region set per service provided to the user and per application program employed by the user, it is feasible to provide the services and functions of the application program matching with the internal reactions or states of the user.

In the Example 1, the guide is performed based on the alignment application so that the sensors are located to the proper specified region by using the captured image of the user, thereby making it easy for the user to conduct the highly accurate alignment to the desirable region in a way that conforms to a usage purpose.

More specifically, in the Example 1, the user terminal 2, based on the dimensions of the respective portions of the head mount apparatus 1, computes the distance between the user's head and the image capturing unit 28 and converts the two-dimensional coordinate system obtained by the image capturing unit 28 into the three-dimensional coordinate system. The user terminal 2 is therefore enabled to convert the image of the image capturing unit 2 8 into the three-dimensional coordinate system at the high accuracy.

In the Example 1, the user terminal 2 has the positions of disposing the sensors on the model of the standard size. The user terminal 2 temporarily obtains the present positions of the sensors based on the image, obtained by the image capturing unit 28, in the two-dimensional coordinate system and the target positions per brain application that are specified in the sensor slider set value management table illustrated in FIG. 10, and displays the objects of the present positions and the target positions of the sensors by making the conversion into the images obtained by the image capturing unit 28 in the two-dimensional coordinate system. The user terminal 2 can therefore guide the user highly accurately.

When using the service or the application program from the second time onward, the user terminal 2 guides the user by superposing the present head image on the head image when the sensors have already been located in the past. The user is therefore enabled to locate the sensors at the present by targeting at the proper locating state having the result of the actual usage in the past, thereby facilitating the adjustment by the user.

<Modified Example>

In the Example, the the preset positions and the target positions of the sensors in the model of the standard size by making the conversion into the three-dimensional coordinate system from the two-dimensional coordinate system obtained by the image capturing unit 28.

It does not, however, mean that the measurement system is limited to these processes described above. For example, the image of the user's head may also be aligned with the model in the two-dimensional coordinate system (XY coordinate system) obtained by the image capturing unit 28. In this case, it may be sufficient that the measurement position per brain application is converted into the two-dimensional coordinate system of the screen from the measurement position in the three-dimensional coordinate system of the brain cortex of the model. The alignment on the image in the two-dimensional coordinate system has a possibility that the accuracy becomes lower than in the Example 1, but the processes by the user terminal 2 are simplified.

[Example 2]

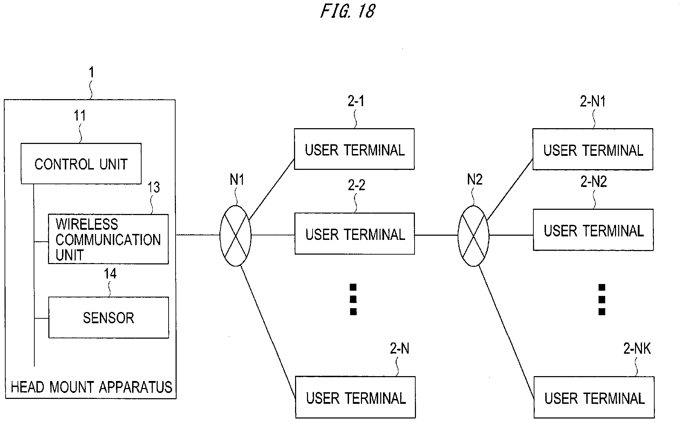

The measurement system according to an Example 2 will hereinafter be described with reference to FIG. 18. In the Example 2, the head mount apparatus 1 exemplified in the Example 1 is connected to a plurality of user terminals 2-1, 2-2, 2-N via the network N1. Components and operations in the Example 2 are the same as those in the Example 1 except a point that the user terminals 2-1, 2-2, 2-N are connected to the same head mount apparatus 1 via the network N1 at the same timing or for the same period.

The measurement system according to the Example 2 may also be configured so that any one of the user terminals 2-1 through 2-N is connected via the network N2 to a plurality of user terminals 2-N1, 2-N2, 2-NK. Herein, the network N1 is a network to which, e.g., the wireless communication unit 13 is connected similarly to FIG. 1. The network N2 is, e.g., the public line network similarly to FIG. 1.

Note that the user terminals 2-1, 2-2, 2-NK may not be necessarily the same type of computers. In the Example 2, the user terminals 2-1, 2-2, 2-NK are, when generically termed, referred to simply as the user terminals 2. In the Example 2, the user wearing the head mount apparatus 1 will hereinafter be called a testee (test subject).

The measurement system having the configuration as in FIG. 18, i.e., the system as an environment configured such that a plurality of members shares the measurement data of the variations of the bloodflow from one testee with each other, is exemplified by a system configured such that reactions of the testee to commercial products, services and other equivalents are transferred to the user terminals 2-1, 2-2, 2-NK when planning, developing or evaluating the commercial products, the services and other equivalents. The reactions to these commercial products and the services can be exemplified by reactions of the testee when operating individual-use equipments, reactions of the testee when looking at fashion items and fashion designs, reactions of the testee when eating and drinking foods, beverages and other equivalents, reactions of the testee when using health-care equipments, massage machines and other equivalent equipments, reactions of the testee when dosed with drugs and medicines, reactions of the testee when watching TV programs, listening to music, looking at advertisements and watching movies and drams, reactions of the testee when reading books, reactions of the testee when performing games and other equivalent applications on electronic equipments, reactions of the testee when enjoying attractions at theme parks, game centers and other equivalent places, reactions of the testee when playing pinballs, slot machines and other equivalent amusements, and reactions of the testee when doing specified excises, taking specified behaviors or performing specified works.

Note that the headmount apparatuses 1 worn on the plurality of testees may also be connected to the user terminals 2-1, 2-2, 2-NK via the networks N1, N2 in the Example 2 similarly to an Example 3 that will be described later on. This is because the plurality of users of the user terminals 2-1, 2-2, 2-N desires to acquire the reactions from the plurality of testees at the same timing or for the same period.

[Example 3]

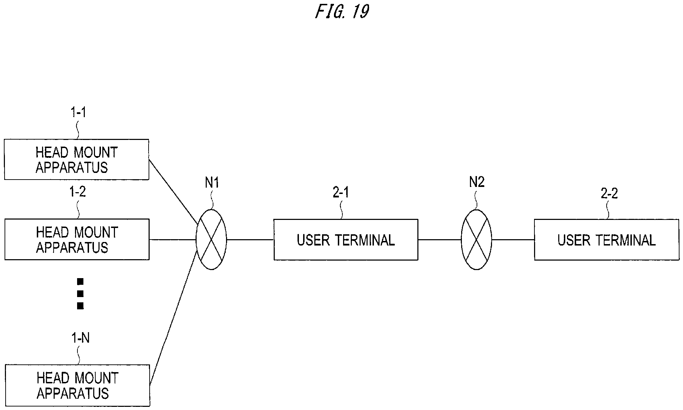

The measurement system according an Example 3 will hereinafter be described with reference to FIG. 19. In the Example 3, a plurality of head mount apparatuses 1-1, 1-2, 1-N each having the same configuration as that of the head mount apparatus 1 exemplified in the Example 1 is connected to a user terminal 2-1 via the network N1. The components and the operations in the Example 3 are the same as those in the Example 1 except a point that the plurality of head mount apparatuses 1-1, 1-2, 1-N is connected to the user terminal 2 via the network N1.

The measurement system in the example 3 may also be configured such that the user terminal 2-1 is connected further to a user terminal 2-2 via the network N2. The networks N1, N2 are the same as those in the examples 1, 2. In the Example 3, the head mount apparatuses 1-1, 1-2, 1-N are, when generically termed, simply referred to as the head mount apparatuses 1.

The measurement system having the configuration as in FIG. 19, i.e., the system as an environment configured to collect the measurement data of the variations of the bloodflow from the plurality of testees, is exemplified by a system configured to transfer reactions of the testees when providing a plurality of persons with the commercial products, the services and other equivalents to the user terminals 2-1, 2-2. Reactions to these commercial products and services can be exemplified by reactions or effects of the testees when receiving learning courses and a variety of seminars at a variety of school and cramming schools, reactions or effects of the testees when conducting specified works, reactions of the testees to services and excises at fitness clubs and other equivalent places, reactions of the testees when provided with services through mass media instanced by TV broadcasts and the Internet.

Drivers,crews,navigators,steersmen and other equivalent operators of, e.g., public transports, airplanes, ships/vessels, trains or buses wear the head mount apparatuses 1, thereby making it possible to monitor physical conditions and variations of the bloodflow rates of the brains of the operators of the public transports, the airplanes, the ships/vessels and the trains. For example, taxi drivers and truck drivers wear the head mount apparatuses 1, thereby making it feasible to monitor physical conditions and variations of the bloodflow rates of the brains of these drivers.

The measurement system according to the Example 3 can monitor training effects in the plurality of persons when trained for (organizing a team) cooperative works, whether activities become aggressive when harmonized and whether sympathetic activities are conducted within the team.

Note that the plurality of user terminals 2-1, 2-2, 2-NK may be connected to the plurality of head mount apparatuses 1-1, 1-2, 1-N via the networks N1, N2 at the same timing or for the same period. This is because the users of the plurality of user terminals 2-1, 2-2, 2-NK desire to acquire the reactions from the plurality of testees at the same timing or for the same period.

In the Example 3, it may be sufficient that the user terminal 2-1 identifies the users as the testees by embedding IDs for identifying the head mount apparatuses 1-1, 1-2, 1-N in a header field of communication data or a user data field (payload field) in the communication data when performing the communications on the network N1 illustrated in FIG. 1. The user terminal 2-2 may also identify the users as the testees by embedding IDs for identifying the head mount apparatuses 1-1, 1-2, 1-N in the user data field (payload field) in the communication data when performing the communications on the network N2.

[Example 4]

(Data ID, Vendor Identifying Information)

In the present embodiment, e.g., when performing the communications on the network N1 illustrated in FIG. 1, the header field in the communication data or the user data field (payload field) in the communication data may receive embeddings of the IDs for identifying the plurality of head mount apparatuses 1, or a maker ID for identifying a regular maker manufacturing the head mount apparatus 1, or a vendor ID for identifying a regular vendor vending the head mount apparatus 1. These IDs described above are desirably stored in the payload field in terms of versatility of the communications. These IDs may also be transferred together with the measurement data of the variations of the bloodflow via the network N2.

The IDs for identifying the head mount apparatuses 1 are embedded in the communication data, thereby facilitating classification of the measurement data per measurement target, which are given from the plurality of head mount apparatuses 1. The maker ID and the vendor ID are embedded in the communication data, thereby enabling the user terminal 2 to eliminate the head mount apparatus 1 that was manufactured or vended in a non-regular manner when running the alignment application or the brain application.

These IDs may be transmitted before transmitting the measurement data to the user terminal 2 from the head mount apparatus 1. For example, the user terminal 2 of the user, when receiving a request for providing the service or the function based on the measurement data of the variations of the bloodflow from the head mount apparatus 1, may acquire the ID from the head mount apparatus 1. The user terminal 2, when acquiring the ID of the head mount apparatus 1, may request the user to input authentication information for authenticating the individual user.

[Example 5]

(Business Model, Pay-As-You-Go (PAYG), Smartphone Application)

As illustrated in FIG. 1, the user terminal 2 is connected to the carrier server 3 via the network N2. The carrier server 3 is connected to the accounting server 4 via the network N2 or the dedicated network N3 for establishing the connection to the computer of the business person.

The carrier server 3 is a computer of the maker of the head mount apparatus 1 or the vendor for providing the brain application. The carrier server 3 manages accounting when using the brain application by providing the brain application to the user.

For instance, the user terminal 2 may be configured to accept a request for providing a fee-charging service on the basis of the measurement data of the variations of the bloodflow rate of the head region, which is detected by the head mount apparatus 1 mounted on the user's head. For example, the user terminal 2, when accepting the request for running the brain application explained in the Examples 1 - 4, may display a query about whether the user consents to the accounting concomitant with running the brain application on the display unit 25. When the user consents to the accounting, it may be sufficient that the user terminal 2 notifies the carrier server 3 of the consent to the accounting, and thereafter provides the service or the function described in the Examples.

On the occasion of obtaining the consent to the accounting, it may be sufficient that the user terminal 2 requests the user to input security information instanced by a predetermined password. It may be sufficient that the carrier server 3, after approving the user with the security information from the user, accepts the consent to the accounting from the user terminal 2. It may be sufficient that the carrier server 3, after accepting the consent to the accounting, requests the accounting server 4 connected to the network N2 or the dedicated network N3 to execute an accounting process.

The accounting server 4 is, e.g., a computer used for the carrier of the public line network (e.g., the network N2) subscribed by the user to manage communications charges of the user. The accounting server 4 may, however, be a computer used for a credit card company previously agreed upon with the user via an accounting management web page on the carrier server 3 to manage a credit card usage amount of the user. In this case, the user previously provides the carrier server 3 with a card number of the credit card issued by the credit card company. However, the user may provide the carrier server 3 with the card number of the credit card issued by the credit card company per accounting approval from the user terminal 2. The configuration described above enables the measurement system to provide the user with the variety of services or functions on the basis of the measurement data of the variations of the bloodflow of the user's head, and to conduct the accounting matching with needs of the user.

The accounting server 4, upon completion of the accounting process, transmits accounting process complete (settled) notification to the carrier server 3. It may be sufficient that the carrier server 3, upon receiving the accounting process complete (settled) notification from the accounting server 4, notifies the user terminal 2 that the accounting process is completed (settled) . It may also be sufficient that the user terminal 2 provides the user with the variety of services explained in the Examples 1 - 4 after completing the accounting process.

[Example 6]

(Business Model, Pay-As-You-Go (PAYG), Web Site)

In the Example 5, the user terminal 2 accepts the request for providing the fee-charging service from the user, and provides the service to the user by requesting the carrier server 3 for the accounting process. The carrier server 3 may also provide with such a process at, e.g., a web site.

In an Example 6, the dedicated brain application installed into the user terminal 2 runs based on a user's operation so that a browser accesses the web site. The carrier server 3 providing the web site accepts the request for providing the fee-charging service, based on the measurement data of the variations of the head bloodflow rate detected by the head mount apparatus 1 mounted on the user's head.

Also when the carrier server 3 accepts the request for providing the fee-charging service, a query about whether the accounting is approvedmay be displayed on the display unit 25 through the brain application or the browser of the user terminal 2. When the user approves the accounting, it may be sufficient that the carrier server 3 provides the service or the function explained in the examples 1 - 4 described above.

On the occasion of the approval of the accounting, it may be sufficient that the carrier server 3 requests the user to input the security information instanced by the predetermined password. It may be sufficient that the carrier server 3 accepts the accounting approval from the user terminal 2 after the user has been approved by the security information given from the user. After accepting the accounting approval, the carrier server 3 may also simply request the accounting server 4 connected to the network N2 or the dedicated network N3 to execute the accounting process.

The carrier server 3, upon receiving the accounting process complete notification from the accounting server 4, requests the user terminal 2 to transmit the measurement data, and acquires the measurement data of the variations of the head bloodflow rate detected from the user. It may be sufficient that the carrier server 3 provides the user with the variety of services based on the measurement data at the web site and other equivalent sites.

[Example 7]

In the Examples 5 and 6, whenever receiving the request for providing the fee-charging service from the user, the carrier server 3 requests the accounting server 4 to execute the accounting process. A process as a substitute for this process is that the carrier server 3 executes the accounting process when downloading the brain application onto the user terminal 2, and thereafter may enable the user to run the brain application on the user terminal 2 charge-free.

In an Example 7, the carrier server 3 accepts, from the user terminal 2, a request for downloading a fee-charging application program for processing the measurement data transferred from the head mount apparatus 1 to detect the variations of the bloodflow rate of the head by being mounted on the user's head at the web site.

The carrier server 3, when accepting the fee-charging download request from the user terminal 2, transmits the execution of the accounting process about the fee-charging service to the accounting server 4 on the network. In this case also, the carrier server 3 may request the user to input the security information instanced by the predetermined password from the user terminal 2 on the web site. It may be sufficient that the carrier server 3, after approving the user with the security information inputted from the user, accepts the accounting approval from the user terminal 2. It may be sufficient that the carrier server 3, after accepting the accounting approval, requests the accounting server 4 connected to the network N2 of the dedicated network N3 to execute the accounting process. It may be sufficient that the carrier server 3, upon receiving the accounting process complete notification from the accounting server 4, transmits the application program to the user terminal 2.

[Modified Example]

Note that in the Example 7 (and an Example 8 that follows), the carrier server 3 executes the accounting process when using the brain application after installing the brain application or when downloading the brain application. It does not, however, mean that the measurement system is limited to the process in the Example 7 or the Example 8. In place of this process, the carrier server 3 may execute a process of decreasing some sort of a monetary value already possessed by the user as a counter value for using or downloading the brain application. To be specific, such a method is available that the user is assumed to previously hold a point equivalent to the monetary value, and this point is consumed when the user uses the present brain application after being installed or when the user downloads the brain application. Herein, the point equivalent to the monetary value may also be a point previously acquired and exchanged with an item having the monetary value by the user, and may also be a point having a monetary substitute value that is accumulated by using the user terminal 2 for a long period and using other applications. The carrier server 3 illustrated in FIG. 1 may accumulate and manage the monetary substitute values. The accounting server 4 cooperating with the carrier server 3, and another server for managing the points may also accumulate and manage the monetary substitute values.

[Example 8]

(Behavior Data, Environment Information)

In the measurement system exemplified in each of the Examples 1 - 7, the user terminal 2 may acquire other physical quantities together with the measurement data of the variations of the user' s bloodflow measured by the head mount apparatus 1, and may provide the services based on a combination of the variations of the bloodflow rate and other physical quantities. The server instanced by the carrier server 3 may acquire these physical quantities from the user terminal 2, and may provide the services based on the measurement data of the variations of the bloodflow, in which case the carrier server 3 and other equivalent servers may provide the services based on the combination of the variations of the bloodflow rate and other physical quantities. The user terminal 2 or the carrier server 3 (which will hereinafter be referred to as the user terminal 2 or another equipment) may provide services based on a combination of the variations of the bloodflow rate and history information about a history of user's behaviors, e.g., a history of accesses to information providing sources on the Internet.

For acquiring these other physical quantities and the history information, the user terminal 2 may include at least one of means to detect a visual line of the user, means to detect a voice of the user, information input operation detecting means, means to detect a shift, a speed, an acceleration or an angular speed of the position of holding the user terminal 2 in hand, positioning means, means to acquire environment information containing at least one of a whether, a noise, a temperature, a humidity, an air pressure and a water pressure, and means to acquire the history of accesses to the information providing sources on the Internet. It may be sufficient that the carrier server 3 acquires these physical quantities or the history information from the user terminal 2.

For example, the user terminal 2 can determine the internal states pertaining to the activity states of the brain, the intentions and the actions of the user more exactly owing to the combinations of the variations of the bloodflow rate of the user and the objects existing along the direction of the visual line on the display unit 25 or variations of the visual line. Note that it may be sufficient that the user terminal 2 determines the direction of the visual line from the eye positions in the face image of the user and from where iris/pupil and sclera of each eye are disposed. The same is applied to a case of combining variations and loudness of the voice of the user, and words obtained from voice recognition and uttered from the user with the variations of the bloodflow rate. It may be sufficient that the user terminal 2 acquires the variations and the loudness of the voice of the user from a microphone provided in the user terminal 2. It may also be sufficient that the user terminal 2 carries out the voice recognition based on the user's voice acquired from the microphone.

It may be sufficient that the user terminal 2 acquires the information to be inputted by the user, trembles and shakes of fingers when user perform inputting onto a touch panel and a touch pad, the shift, the speed, the acceleration or the angular speed of the position of holding the user terminal 2 in hand, and combines these items of data with the variations of the bloodflow rate of the user. It may also be sufficient that the user terminal 2 acquires the environment information containing at least one of positional information of the GPS and other positioning means, the weather, the noise, the temperature, the humidity, the air pressure and the water pressure, and and combines these items of data with the variations of the bloodflow rate of the user. The user terminal 2 may further acquire the variations of the bloodflow rate of the user from the head mount apparatus 1 when the user accesses the information providing sources on the Internet. It may further be sufficient that the user terminal 2 combines the history of the accesses to the information providing sources on the Internet with the variations of the bloodflow rate of the user.

[Example 9]

(Auto-Pickup of Interested Target Content)

Described next is an applied example to a system configured to pick up a content in which the user has an interest by using the measurement system according to the Example 1. Specifically, the Example 9 exemplifies, in addition to the measurement system according to the Example 1, a system configured such that the user terminal 2 acquires the direction of the visual line of the user or a position, within the user terminal 2, based on a pointer instanced by the finger and other equivalent regions of the user and displays a content corresponding to this position when the variations occur in the bloodflow rate of the user that is measured by the head mount apparatus 1. This system enables the user terminal 2 to automatically pick up the user's interested target determined based on the variations of the bloodflow rate of the user, thereby eliminating a necessity for actively selecting the interested target.

Herein, "the occurrence of the variations in the bloodflow rate" implies a case that the variation of the bloodflow rate per time or a derivative of the variation of the bloodflow rate is larger than a fixed threshold value being set based on an empirical rule, and encompasses a case of causing a minute change in variation of the bloodflow rate corresponding to the state. The information processing apparatus (including the CPU 21 and the memory 22 illustrated in, e.g., FIG. 1) within the user terminal 2 determines the direction of the visual line from the eye positions in the face image of the user and from where the iris/pupil and the sclera of each eye are disposed when the variation occurs in the bloodflow rate of the user, and specifies a target portion of the visual line from within articles and information displayed on the user terminal 2, based on a position of the user terminal 2 and a relative position of the eyes of the user.

The information processing apparatus of the user terminal 2 may also specify, in place of the information about the direction of the visual line, a position of the pointer pointing the position of the finger or another equivalent region of the user when the variation occurs in the bloodflow of the user, based on the information about the direction of the visual line. This is because the pointer position is a position pointed by the user's finger when the variation occurs in the bloodflow rate, and is therefore made fictitious with a portion in which the user has the interest. Herein, the position of the pointer pointing the position of the user's finger connotes, e.g., a position of the finger that contacts the touch panel and a pointer position on the screen of the display unit 25 by using the touch pad when the operation unit 26 of the user terminal 2 illustrated in FIG. 1 includes the touch panel, the touch pad and other equivalent devices.

The portion in which the user has the interest involves using, as described above, any one of the visual line of the user, the contact position of the user's finger on the screen and the pointer position on the screen, and may also be determined comprehensively by combining the visual line and these positions together. To be specific, final coordinates are specified by weighting, corresponding to a degree of importance, coordinates obtained based on the visual line of the user within the display range of the user terminal 2, and coordinates obtained based on the contact position of the user's finger on the screen or the pointer position on the screen within the display range of the user terminal 2. For example, the direction of the visual line of the user is ambiguous as the case may be in terms of the relative position to the user terminal 2. Specifically, the target portion of the visual line of the user is determined to be beyond the range of the display device of the user terminal 2 as the case may be in terms of the direction of the visual line of the user and the relative position to the user terminal 2. Thus, the information processing apparatus determines that the position indicated by the visual line of the user is ambiguous or unreliable inclusively of other items of information, in which case the information processing apparatus sets, low or to "0", the weight of the coordinates based on the direction of the visual line of the user. Especially hereat, supposing that the pointer within the user terminal 2 keeps moving until just before the variation occurs in the bloodflow rate of the user, the portion in which the user has the interest may be specified from only the pointer by setting the weight of the coordinates to "0", based on the direction of the visual line of the user. This is because there is a possibility that the user intentionally changes the pointer position by the finger. While on the other hand, when the pointer position does not change just before the occurrence of the variations of the bloodflow rate of the user, such a possibility is high that the user does not intentionally move the pointer, and hence the visual line of the user is emphasized by setting, low or to "0", the weight of the coordinates based on the pointer position.

The user terminal 2 can specify the interested portion in which the user has a particular interest by, e.g., separately displaying the thus specified information within the user terminal 2 without any necessity for manually copying and pasting the specified information.

When the measurement system is further applied to a search system, a content pertaining to the interested portion acquired by the measurement system is utilized as auxiliary information for a next search, thereby enabling the user to perform searching much closer to the content in which the user will have the interest.

[Example 10]

(Emergency Call System)

Next, a system for automatically performing an emergency call will be described. In the system of the Example 1, the user terminal 2 according to an example 10 further includes means to determine that a life of the person is at risk due to the variations of the bloodflow rate of the user, and means to transfer information to a predetermined terminal or computer (which will hereinafter be simply termed the terminals) upon occurrence of the risk. Herein, the means to determine that the risk occurs in the life of the person is specifically the means to make this determination when the information processing apparatus (including the CPU 21 and the memory 22 illustrated in FIG. 1) compares data obtained by measuring blood quantities, necessary for maintaining the life of the person, of predetermined regions within the brain on a region-by-region basis with required blood quantities based on the variations of the bloodflow rate of the user, which is measured by the head mount apparatus 1, and detects that the bloodflow rate of the user becomes lower than a value of the data.

As the information to be transferred to the terminals from the user terminal 2, only the information indicating "being at risk" may be transferred; the variations of the present bloodflow rate or information about the variations of the present bloodflow rate may also be transferred; and the information indicating "being at risk" and the variations of the present bloodflow rate or the information about the variations of the present bloodflow rate may further be transferred.

The terminals connote terminals equipped within emergency medical institutes capable of emergency medical services or terminals connectable to these medical institutes. In other words, it may be sufficient that the information is transferred to the terminals belonging to the institutes capable of taking the appropriate medical services upon the occurrence of the risk to the life of the person.

The foregoing data obtained by measuring the blood quantities, necessary for maintaining the life of the person, of the predetermined regions within the brain on the region-by-region basis may be retained as a data set within the information processing apparatus, and may also be acquired according to the necessity via the network N2 from another computer, e.g., the carrier server 3 linked to the network and other computers of medical institutes, public institutes, research institutes and universities.

In the Example 10, upon the occurrence of the risk to the user's life, the information indicating the risk is transferred to the emergency medical institute for the emergency medical services via the user terminal 2, thereby enabling taking measures of performing an appropriate medical service instanced by dispatching a staff member of the emergency medical institute to user's home or calling the user terminal 2 for a making query about a user's state.

[Example 11]

(GPS)

An example 11 exemplifies a measurement system configured such that the user terminal 2 according to the Example 10 further includes a GPS (Global Positioning System), and position information obtained by the GPS can be transmitted to the terminals of the Example 10 at predetermined time. The predetermined time encompasses a time when the variations of the bloodflow rate occur in a specified state, in addition to when the risk occurs in the user's life. The information can be thereby transmitted to the terminals in the Example 10 just when the preset variation of the bloodflow rate occurs even before the risk to the life arises.

The information transmitted to the terminals may be may be any one of the information indicating "being at risk", the information indicating the bloodflow rate of the brain and the position information, or may also be a combination of these items of information.

The Examples 10 and 11 can be utilized by the users, e.g., persons performing activities in dangerous places as instanced by mountaineers, skiers, explorers and soldiers (armies) in addition to aged persons and patients.

[Example 12]

(Operation on User Terminal from User's Viewpoint: Brain Training)

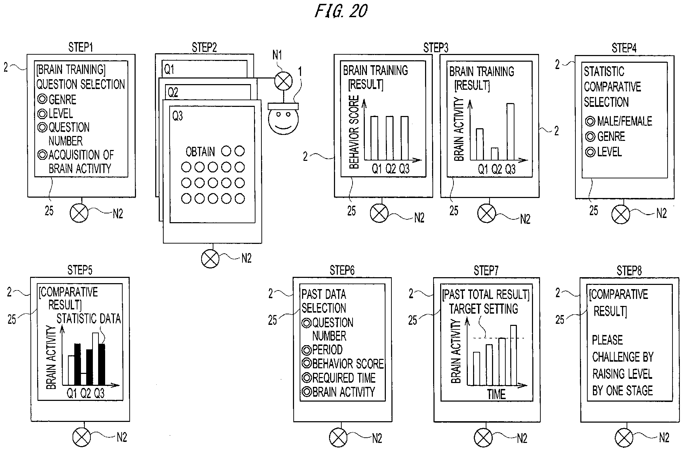

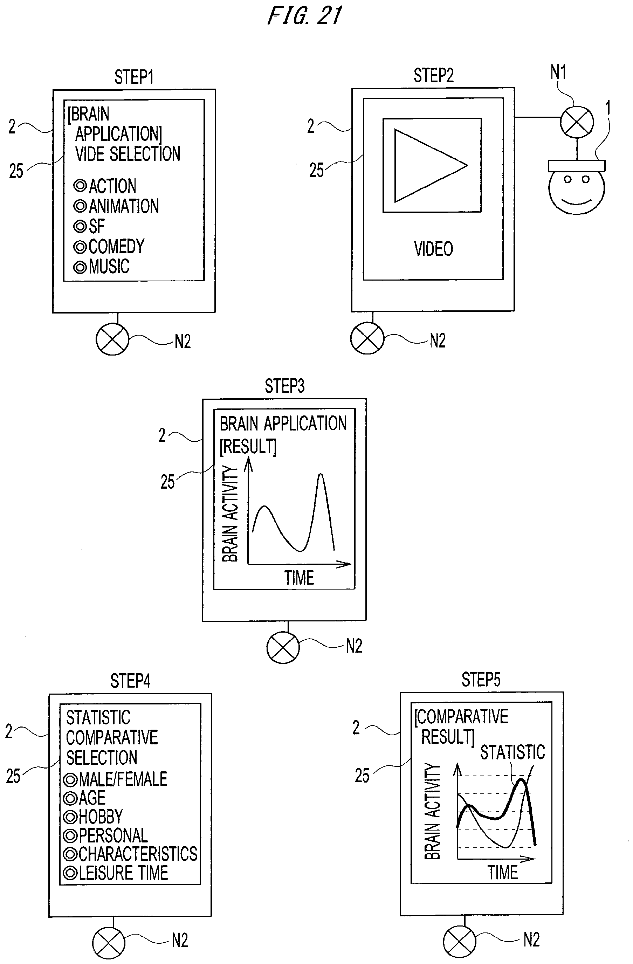

In the measurement system illustrated in each of FIGS. 1 - 8, an operational example of the user terminal 2 will be described with reference to FIGS. 20 and 21. FIGS. 20 and 21 illustrate abstractions of the user terminal 2, the display unit 25, the head mount apparatus 1, the network N1 and the network N2 for the public line communications in FIG. 1. FIGS. 20 and 21 also illustrate examples of operating procedures of the user in a sequential order from Step1.

An Example 12 will omit the same repetitive explanations already made in the Examples 1 - 8. A description of the Example 12 will start from a point that the brain application has already been downloaded into the user terminal 2, and the calibration of the head mount apparatus 1 has been completed. Hereinafter, the user terminal 2 runs an application program for brain training (which will hereinafter simply be termed the brain training) as one of the brain applications.

Step 1 : When the user terminal 2 starts up a brain training application in accordance with a user's operation, a question selection (question selecting screen) is displayed. The user selects a desired genre (e.g., calculation, Chinese character, graphics and puzzle), a level (entry level, an intermediate level and an advanced level), a question number and a brain activity to thereby start the brain training, and the user terminal 2 starts importing the measurement data representing the variations of the bloodflow rate of the user via the network N1 from the head mount apparatus 1.

Step 2: A question Q1 selected on the question selecting screen is displayed, and the user performs an operation based on an instruction of the displayed question. The normal brain training program saves, as a behavior score, a score achieved at this time by the user through the brain training. On the other hand, the brain training application according to the Example 12 converts the data, imported via the network N1, of the variations of the bloodflow rate into a score of the brain activity and saves the converted score in the user terminal 2 in parallel with performing the brain training of the user. It may be sufficient that an algorithm for converting the brain activities into scores from the variations of the bloodflow rate involves applying existing cases (refer to, e.g., Non-Patent Documents 1 and 2). This algorithm will, however, keep being improved from now on. In other words, the providers of the brain applications will keep incorporating the most updated algorithms into the brain applications from now on. After similarly iteratively implementing the questions Q2, Q3 and scheduled questions in continuation and just when completing all the scheduled questions, an evaluation of the brain training is completed. Finished hereat is the importation of the measurement data of the variations of the bloodflow rate of the user into the user terminal 2 via the network N1 from the head mount apparatus 1.

Step 3: A result of the brain training is displayed. Normally, the result of the brain training is displayed as the behavior score per question. On the other hand, in the Example 11, the result of the brain training can be displayed as a score of the brain activity per question. This is characteristic of the present measurement system capable of running the brain training application on the user terminal 2, and displaying the variations of the bloodflow rate of the head simply as the scores of the brain activities.

Step 4: One of methods of facilitating comprehension of the scores of the brain activities of the user himself or herself is a comparative method using static data stored in a database on the network N2. The user can compare the score of the user himself or herself with the statistic data by inputting a male or female gender, an age, a genre and a level on a statistic comparative selection screen of the brain training application.

The user can access the database from on the screen of the brain application. The user selects, e.g., "a 40-year-old female, genre: calculation, level: beginner", and is thereby enabled to acquire the statistic data of this condition via the network N2. Note that a server of a vendor of the brain training application or the carrier server 3 illustrated in FIG. 1 is assumed as a provider of the present database for the time being. However, when the measurement system will be broadly effectively utilizable, such a possibility exists that the provider of the measurement system will be diversified to a variety of providers.

Step 5: The brain training application displays the comparison between the score of the brain activity of the user and the statistic data acquired by making the statistic comparative selection. Information to be displayed represents a relative comparison in any case, and simply facilitates understanding a value thereof.

Step 6: The user saves the self core of the brain activity and can easily invoke the score afterward. The user can select a question number of the brain training, a period, a behavior score, a required time and a brain activity score from on a past data selection screen.

Step 7: The user can display totaled results in the past in accordance with an instruction of selecting the past data. The user has hitherto undergone the training based on the self behavior score, and the measurement system, however, enables the user to convert the self brain activity into the score (convert the variation value of the bloodflow rate into the score), and to undergo the training based on the score of the brain activity. The user sets target set values of the brain activities, and can display these values in graph.

Step 8: When the user attains the target set value of the brain activity, the brain application can display a message for prompting the user to challenge a higher level of the brain training from within other brain applications.

The Example 12, though having exemplified the brain training, can be developed to convert the brain activities about videos (an action video, an animation video, an SF (Scientific Fiction) video, a comedy video and a music video) into scores, and can be also developed to undergo cognitive training and restraint training. The user sets a target value of the brain activity, and can perform feedback training by using the variations of the self bloodflow rate toward attaining the target value (see FIG. 21).

FIG. 21 is a diagram illustrating processes of the brain application configured to enable states of the brain activities to be monitored when the user watches the video. Similarly to the case in FIG. 20, it is assumed that the brain application is already downloaded into the user terminal 2, and the calibration of the head mount apparatus 1 is already completed.

Step 1: The user selects, e.g., the video from on a video selection screen of the brain application. FIG. 21 illustrate an example of the genre instanced by the action video, the animation video, the SF video, the comedy video and the music video.

Step 2: The user records the data of the bloodflow rate from the head mount apparatus 1 by running the brain application while watching the video selected in Step1 via the display unit 25 and the output unit 27 of the user terminal 2.

Step 3 : The user displays the recorded data of the bloodflow rate.

Step 4: The user is able to access the database of the statistic data from on the screen of the brain application similarly to the case of Step4 in FIG. 20. The user inputs the male/female gender, the age, a hobby, personal characteristics, a leisure time and other items, thereby enabling a comparison between the score of the user himself or herself and the statistic data.

Step 5: The brain training application (the user terminal 2) displays the comparison between the score of the brain activity of the user and the statistic data acquired by making the statistic comparative selection.

Although one example of the operation of the user terminal in terms of the viewpoint of the user has been has been exemplified so far, it can be easily analogically construed by persons skilled in the art to similarly perform the display and the operation of the user terminal in the measurement system and in all of the services described in the present specification, and everything described above is encompassed as respective aspects of the present invention in the scope of the disclosure of the present embodiment.

[Others]

The measurement system disclosed in the present embodiment has features in the respective aspects, and is enabled to grasp various categories of configurations, operations and effects. For example, the categories of the configurations will be exemplified as follows and may be grasped as:

> (a) the whole measurement system illustrated in FIG. 1;
> (b) the single head mount apparatus 1;
> (c) the single user terminal 2;
> (d) the application program, e.g., the alignment application, the brain application and other equivalent applications that are run on the user terminal 2;
> (e) the method executed by the user terminal 2;
> (f) the single carrier server 3;
> (g) the application program, e.g., the alignment application, the brain application and other equivalent applications that are run on the carrier server 3;
> (h) the method executed by the carrier server 3; and
> (i) the system including at least one of the head mount apparatus 1, the user terminal 2, the carrier server 3 and the accounting server 4, and the method executed by the system.

[Non-Transitory Recording Medium]

The program described in the embodiment can be recorded on a non-transitory recording medium readable by the computer and other equivalent apparatuses, and the program on the non-transitory recording medium can be read and run by the computer.

Herein, the non-transitory recording medium readable by the computer and other equivalent apparatuses connotes a non-transitory medium capable of accumulating information instanced by data, programs and other equivalent information electrically, magnetically, optically, mechanically or by chemical action, which can be read from the computer and other equivalent apparatuses. These non-transitory recording mediums are exemplified by a flexible disc, a magneto-optic disc, a CD-ROM, a CD-R/W, a DVD, a Blu-ray disc, a DAT, an 8 mm tape, and a memory card like a flash memory. A hard disc, a ROM (Read-Only Memory) and other equivalent recording mediums are given as the non-transitory recording mediums fixed within the computer and other equivalent apparatuses. Further, an SSD (Solid State Drive) is also available as the non-transitory recording medium removable from the computer and other equivalent apparatuses and also as the non-transitory recording medium fixed within the computer and other equivalent apparatuses.

[Brief Description of the Reference Numerals and Symbols]

| | |
|---|---|
| 1 | head mount apparatus |
| 2 | user terminal |
| 3 | carrier server |
| 4 | accounting server |
| 11 | control unit |
| 13 | wireless communication unit |
| 21 | CPU |
| 22 | memory |
| 23 | wireless communication unit |
| 24 | public line communication unit |
| 25 | display unit |
| 26 | operation unit |
| 27 | output unit |
| 28 | image capturing unit |
| 29 | positioning unit |
| 2A | physical sensor |
| 100 | base member |
| 102 | battery box |
| 111, 112 | housing |
| 112, 122 | knobs |
| 103, 113, 123 | marker |
| 114, 124 | apertures |
| 115, 125 | sensor |

Claims

1. A measurement system comprising:

   a head mount apparatus including:

   detection means to detect a variation of a bloodflow rate of a head of a user, the detection means being mounted on the head; and
   transfer means to transfer a detection value of the detection means to a predetermined transfer destination; and

   an information processing apparatus including:

   receiving means to receive the detection value transferred from the transfer means; and
   service providing means to provide a service to the user, based on the received detection value.

2. The measurement system according to claim 1, wherein the head mount apparatus further includes locating means to locate the detection means at a specified region of the head.

3. The measurement system according to claim 2, wherein the specified region is a head surface corresponding to a frontal lobe.

4. The measurement system according to any one of claims 1 through 3, wherein the transfer means transfers the detection value to the transfer destination via a wireless communication path.

5. The measurement system according to any one of claims 1 through 4, wherein the information processing apparatus is a mobile terminal including public wireless communication means to access a public wireless network.

6. The measurement system according to claim 2, wherein the information processing apparatus further includes means to support an adjustment of the locating means for the user to locate the detection means at the specified region corresponding to a service or classification of the service provided by the service providing means.

7. The measurement system according to claim 6, wherein the information processing apparatus further includes:

   means to acquire a present head image of the user wearing the head mount apparatus;
   means to acquire saved head images of the user when the user is provided with the services in the past; and
means to support the adjustment of the locating means for the user to locate the detection means at the specified region corresponding to the service or the classification of the service, based on the present head image and the saved head images.

8. The measurement system according to any one of claims 1 through 7, wherein the transfer means transfers the detection value to a plurality of information processing apparatuses.

9. The measurement system according to any one of claims 1 through 8, wherein the receiving means receives the detection values transferred respectively from the plurality of head mount apparatuses, and the service providing means provides services to a plurality of users wearing the head mount apparatuses.

10. The measurement system according to any one of claims 1 through 9, wherein the head mount apparatus further includes:

    means to retain identifying information used for the information processing apparatus to identify the plurality of head mount apparatuses; and
    means to hand over the identifying information to the information processing apparatus when the transfer means transfers the detection value.

11. The measurement system according to claim 10, wherein the information processing apparatus further includes means to accept an input of authentication information for authenticating the user of the head mount apparatus identified by the identifying information upon the handover of the identifying information.

12. The measurement system according to any one of claims 1 through 11, wherein the head mount apparatus includes means to hand over validity information for determining whether a self maker or vendor is valid or not, to the information processing apparatus,
    the information processing apparatus includes means to determine whether the maker or vendor of the head mount apparatus is valid or not, based on the validity information acquired from the head mount apparatus, and
    the service providing means restricts the service from providing, corresponding to a result of the determination.

13. The measurement system according to any one of claims 1 through 12, wherein the information processing apparatus includes at least one of

(a) means to detect a visual line of the user,
(b) means to detect a voice,
(c) means to detect an information input operation,
(d) means to detect a shift, a speed, an acceleration or an angular speed of a position of holding the information processing apparatus in hand,
(e) positioning means,
(f) means to acquire environment information containing at least one of (I) a weather, (II) a noise, (III) a temperature, (IV) a humidity, (V) an air pressure and (VI) a water pressure, and (g) means to acquire a history of accesses to an information provider on the Internet.

14. The measurement system according to any one of claims 1 through 13, wherein the service includes at least one of

(a) providing the user with information pertaining to activity states of a brain of the user,
(b) displaying an image,
(c) providing the user with a physical effect containing at least one of (I) a sound, (II) a voice, (III) a vibration and (IV) light,
(d) providing information to a participant participating in a present activity of the user,
(e) controlling a device or equipment being currently used by the user, and
(f) transmitting information to a device or equipment cooperating with the device being currently used by the user.

15. The measurement system according to claim 14, wherein the information pertaining to the activity of the brain of the user contains at least one of

(a) information representing a present state based on a comparison with evaluations, accumulated in the past, of the user,
(b) information containing at least one of user's correlation and comparison with information of other human bodies,
(c) information containing at least one of user's determination and instruction about the information of other human bodies,
(d) information containing at least one of a correlation and a comparison with a physical condition of the user,
(e) information containing at least one of a determination and an instruction about the physical condition of the user,
(f) information containing at least one of a correlation and a comparison with a mental state of the user,

(g) information containing at least one of a determination and an instruction about the mental state of the user, and
(h) information provided on the Internet.

16. A head-mounted device comprising:

mounting means to have marks used for alignment with a reference position of the head when mounted on the head of the user;
detection means to detect a variation of a bloodflow rate of the head in a state of being already aligned with the reference position; and
transfer means to transfer a detection value of the detection means to a predetermined transfer destination.

17. A program for making a computer execute:

a receiving step of receiving a detection value transferred from a head mount apparatus mounted on a head of a user and detecting a variation of a bloodflow rate of the head; and
a service providing step of providing a service to the user, based on the received detection value.

18. A program for making a computer execute:

a step of accepting a request for providing a fee-charging service based on a detection value of a variation of a bloodflow rate of a head, the variation being detected by a head mount apparatus mounted on the head of a user;
a step of instructing a server on a network to execute an accounting process for the fee-charging service upon accepting the fee-charging service providing request; and
a service providing step of providing the user with the fee-charging service after completing the accounting process.

19. A service providing method by which a computer executes:

a step of accepting a request for providing a fee-charging service based on a detection value of a variation of a bloodflow rate of a head from an information processing apparatus of a user, the variation being detected by a head mount apparatus mounted on the head of the user;
a step of instructing an accounting server on a network to execute an accounting process for the fee-charging service upon accepting the fee-charging service providing request from the information processing apparatus;
a step of acquiring the detection value from the information processing apparatus; and

a service providing step of providing the fee-charging service.

20. A service providing method by which a computer executes:

a step of accepting, from an information processing apparatus of a user, a request for a fee-charging download of an application program for processing a detection value of a variation of a bloodflow rate of a head, the variation being detected by a head mount apparatus mounted on the head of the user;
a step of transmitting, to an accounting server on a network, execution of an accounting process about the application program upon accepting the fee-charging download request from the information processing apparatus; and
a step of transmitting the application program to the information processing apparatus.

*FIG. 1*

CONTROL UNIT    13

WIRELESS COMMUNICATION UNIT

SENSOR    115

SENSOR    125

HEAD MOUNT APPARATUS

N1

CPU    21    MEMORY    22

WIRELESS COMMUNICATION UNIT    23

PUBLIC LINE COMMUNICATION UNIT    24

DISPLAY UNITBLIC LINE COMMUNICATION UNIT    25

OPERATION UNIT    26

OUTPUT UNIT    27

IMAGE CAPTURING UNIT    28

POSITIONING UNIT    29

PHYSICAL SENSOR UNIT    2A

USER TERMINAL

N2    N3

CARRIER SERVER    3

ACCOUNTING SERVER    4

FIG. 2

*FIG. 3*

FIG. 4

## FIG. 5

NOSE, CENTRAL POSITION GUIDELINE

HEAD FRAME GUIDELINE

BRAIN BLOODFLOW
MEASUREMENT POSITION GUIDE FRAME

EYEBROW LINE

EYE POSITION GUIDELINE

1/3

1/3

1/3

1/3

1/3

1/3

1/5  1/5  1/5  1/5  1/5

FIG. 6

## FIG. 7

FIG. 8

MEASUREMENT POSITION MANAGEMENT TABLE

| No | NAME OF MEASUREMENT REGION | OFFSET COORDINATE (x) | OFFSET COORDINATE (y) | OFFSET COORDINATE (z) | MEASUREMENT RANGE (r) |
|----|------|------|------|------|------|
| 1 | AREA 1 | | | | |
| | | | | | |
| 2 | AREA 59 | | | | |

*FIG. 9*

| No | ITEM | SIZE | OFFSET POSITION (x) | OFFSET POSITION (y) | OFFSET POSITION (z) | MOVABLE RANGE (±mm) |
|---|---|---|---|---|---|---|
| 1 | HEADSET HORIZONTAL LENGTH | | | | | |
| 2 | HEADSET VERTICAL LENGTH | | | | | |
| 3 | MARKER L1 | | | | | |
| 4 | MARKER M1 | | | | | |
| 5 | MARKER R1 | | | | | |
| 6 | SLIDER L1 | | | | | |
| 7 | SLIDER L2 | | | | | |
| 8 | SENSOR SLIDER MOVABLE RANGE L1 | | | | | |
| 9 | SENSOR SLIDER MOVABLE RANGE R1 | | | | | |
| 10 | SENSOR L11 | | | | | |
| 11 | SENSOR L12 | | | | | |
| 12 | SENSOR R21 | | | | | |
| 13 | SENSOR R22 | | | | | |

FIG. 10

| No | APPLICATION TYPE | RIGHT SENSOR | LEFT SENSOR |
|---|---|---|---|
| 1 | ENGLISH DICTIONARY TRAINING | AREA 46 | AREA 46 |
| 2 | READING TRAINING | AREA 46 | AREA 46 |
| 3 | COGNITIVE TRAINING | AREA 46 | AREA 46 |
| 4 | RESTRAINT TRAINING | AREA 45 | AREA 45 |
| 5 | COOPERATIVE TRAINING | AREA 10 | AREA 10 |

## FIG. 11

INPUT IMAGE MEMORY

FACE IMAGE MEMORY

EYE IMAGE MEMORY

NOSE IMAGE MEMORY

MOUTH IMAGE MEMORY

HEADSET IMAGE MEMORY

MARKER IMAGE MEMORY

SENSOR IMAGE MEMORY

EYE 2D COORDINATE MEMORY

NOSE 2D COORDINATE MEMORY

MOUTH 2D COORDINATE MEMORY

HEADSET 2D COORDINATE MEMORY

MARKER 2D COORDINATE MEMORY

SENSOR 2D COORDINATE MEMORY

EYE POSITION MEMORY

NOSE POSITION MEMORY

MOUTH POSITION MEMORY

HEADSET POSITION MEMORY

MARKER POSITION MEMORY

SENSOR POSITION MEMORY

OUTPUT IMAGE MEMORY

~22

## FIG. 12

## FIG. 13

```
        ┌─────────────────────────────┐
        │    IMAGE PROCESSING UNIT     │
        └─────────────────────────────┘
                       │
                       ▼
        ┌─────────────────────────────┐
        │     IMAGE READING MEANS      │ ～ S21
        └─────────────────────────────┘
                       │
                       ▼
        ┌─────────────────────────────┐
        │     FACE DETECTION MEANS     │ ～ S22
        └─────────────────────────────┘
                       │
                       ▼
        ┌─────────────────────────────┐
        │     EYE DETECTION MEANS      │ ～ S23
        └─────────────────────────────┘
                       │
                       ▼
        ┌─────────────────────────────┐
        │     NOSE DETECTION MEANS     │ ～ S24
        └─────────────────────────────┘
                       │
                       ▼
        ┌─────────────────────────────┐
        │    MOUTH DETECTION MEANS     │ ～ S25
        └─────────────────────────────┘
                       │
                       ▼
        ┌─────────────────────────────┐
        │   HEADSET DETECTION MEANS    │ ～ S26
        └─────────────────────────────┘
                       │
                       ▼
        ┌─────────────────────────────┐
        │    MARKER DETECTION MEANS    │ ～ S27
        └─────────────────────────────┘
                       │
                       ▼
        ┌─────────────────────────────┐
        │    SENSOR DETECTION MEANS    │ ～ S28
        └─────────────────────────────┘
                       │
                       ▼
        ┌─────────────────────────────┐
        │             END             │
        └─────────────────────────────┘
```

## FIG. 14

```
( POSITION COMPUTING UNIT )
              |
              v
```

| | |
|---|---|
| COMPUTE MARKER POSITION BY EXTRACTING MARKER COORDINATE INFORMATION AND PIXEL COUNT OF MARKER FROM MARKER IMAGE MEMORY | S31 |

```
              |
              v
```

| | |
|---|---|
| COMPUTE EYE POSITION IN MARKER AND EYE COORDINATES AND MARKER POSITION | S32 |

```
              |
              v
```

| | |
|---|---|
| COMPUTE NOSE POSITION IN MARKER AND NOSE COORDINATES AND MARKER POSITION | S33 |

```
              |
              v
```

| | |
|---|---|
| COMPUTER MOUTH POSITION IN MARKER AND MOUTH COORDINATES AND MARKER POSITION | S34 |

```
              |
              v
```

| | |
|---|---|
| COMPUTER SENSOR SLIDER POSITION IN MARKER AND SENSOR SLIDER COORDINATES AND MARKER POSITION | S35 |

```
              |
              v
```

| | |
|---|---|
| COMPUTER SENSOR POSITION FROM SENSOR SLIDER POSITION AND SENSOR MOVABLE RANGE OF HEADSET STRUCTURE MANAGEMENT TABLE | S36 |

```
              |
              v
         (   END   )
```

*FIG. 15*

```
        ┌─────────────────────────────┐
        │   IMAGE SYNTHESIZING UNIT    │
        └─────────────────────────────┘
                      │
                      ▼
┌──────────────────────────────────────────────┐
│  ACQUIRE INPUT IMAGE, COPY INPUT IMAGE TO      │
│  OUTPUT IMAGE, AND DISPLAY SENSOR POSITION     │
│  INFORMATION AND BRAIN BLOODFLOW               │─── S61
│  MEASUREMENT SENSOR POSITION INFORMATION       │
│  PER APPLICATION IN SUPERPOSITION              │
└──────────────────────────────────────────────┘
                      │
                      ▼
        ┌─────────────────────────────┐
        │            END              │
        └─────────────────────────────┘
```

## FIG. 16

1

28

IMAGE CAPTURING UNIT

CPU

HEAD MOUNT POSITION ADJUSTMENT APPLICATION

21

25

DISPLAY UNIT

FIRST-TIME ADJUSTMENT INFORMATION STORAGE AREA

IMAGE CAPTURED AT FIRST-TIME ADJUSTMENT

22

HEAD MOUNT POSITION INFORMATION STORAGE AREA

## FIG. 17

```
                    ┌─────────────┐
                    │    START    │
                    └──────┬──────┘
                           ▼
┌──────────────────────────────────────────────────┐
│      MOUNT HEAD MOUNT APPARATUS ON HEAD           │────S71
└──────────────────────┬───────────────────────────┘
                       ▼
┌──────────────────────────────────────────────────┐
│  START UP HEAD MOUNT POSITION ADJUSTMENT APPLICATION │──S72
└──────────────────────┬───────────────────────────┘
                       ▼
┌──────────────────────────────────────────────────┐
│           CAPTURE IMAGE OF TESTEE                 │────S73
└──────────────────────┬───────────────────────────┘
                       ▼
┌──────────────────────────────────────────────────┐
│  ACQUIRE "FIRST-TIME ADJUSTMENT CAPTURED IMAGE"   │
│  FROM STORAGE AREA                                │────S74
└──────────────────────┬───────────────────────────┘
                       ▼
┌──────────────────────────────────────────────────┐
│  ACQUIRE "IMAGE CAPTURED BY CAMERA" FROM IMAGE    │
│  CAPTURING UNIT                                   │────S75
└──────────────────────┬───────────────────────────┘
                       ▼
┌──────────────────────────────────────────────────┐
│  DISPLAY "IMAGE CAPTURED BY CAMERA" AND "FIRST-TIME │
│  ADJUSTMENT CAPTURED IMAGE" TRANSPARENTLY IN      │────S76
│  SUPERPOSITION ON DISPLAY                         │
└──────────────────────┬───────────────────────────┘
                       ▼
┌──────────────────────────────────────────────────┐
│  EXTRACT DIFFERENTIAL IMAGE OF "IMAGE CAPTURED BY │
│  CAMERA" AND "FIRST-TIME ADJUSTMENT CAPTURED IMAGE" │──S77
└──────────────────────┬───────────────────────────┘
                       ▼
```

IS DIFFERENTIAL IMAGE AREA SIZE WITHIN THRESHOLD VALUE? — S78

NO

YES

MOUNT POSITION ADJUSTMENT INSTRUCTION — S79

RECORD HEAD MOUNT POSITION INFORMATION IN HEAD MOUNT POSITION INFORMATION STORAGE AREA — S80

CALIBRATION COMPLETE INSTRUCTION — S81

END

*FIG. 18*

FIG. 19

| 1-1 |
| HEAD MOUNT APPARATUS |

| 1-2 |
| HEAD MOUNT APPARATUS |

| 1-N |
| HEAD MOUNT APPARATUS |

N1

| 2-1 |
| USER TERMINAL |

N2

| 2-2 |
| USER TERMINAL |

## FIG. 20

## FIG. 21

STEP1

2

25

[BRAIN
 APPLICATION]
VIDE SELECTION

◎ACTION
◎ANIMATION
◎SF
◎COMEDY
◎MUSIC

N2

STEP2

N1

1

2

25

▷

VIDEO

N2

STEP3

2

25

BRAIN APPLICATION
[RESULT]

BRAIN ACTIVITY

TIME

N2

STEP4

2

25

STATISTIC
COMPARATIVE
SELECTION
◎MALE/FEMALE
◎AGE
◎HOBBY
◎PERSONAL
◎CHARACTERISTICS
◎LEISURE TIME

N2

STEP5

2

25

[COMPARATIVE
RESULT]
STATISTIC

BRAIN ACTIVITY

TIME

N2

## INTERNATIONAL SEARCH REPORT

| International application No. |
| --- |
| PCT/JP2015/083035 |

**A. CLASSIFICATION OF SUBJECT MATTER**

*G06Q50/22*(2012.01)i, *A61B10/00*(2006.01)i, *G06Q50/10*(2012.01)i

According to International Patent Classification (IPC) or to both national classification and IPC

**B. FIELDS SEARCHED**

Minimum documentation searched (classification system followed by classification symbols)
G06Q50/22, A61B10/00, G06Q50/10

Documentation searched other than minimum documentation to the extent that such documents are included in the fields searched
Jitsuyo Shinan Koho       1922-1996   Jitsuyo Shinan Toroku Koho   1996-2016
Kokai Jitsuyo Shinan Koho   1971-2016   Toroku Jitsuyo Shinan Koho   1994-2016

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y<br>A | JP 2006-305334 A  (Advanced Telecommunications Research Institute International),<br>09 November 2006 (09.11.2006),<br>paragraphs [0036], [0037]<br>(Family: none) | 1-5,8-11,<br>13-20<br>12<br>6-7 |
| Y<br>A | JP 2008-48210 A  (Konica Minolta Business Technologies, Inc.),<br>28 February 2008 (28.02.2008),<br>paragraphs [0093], [0114]<br>& US 2008/0072052 A1 | 12<br>1-11,13-20 |
| A | JP 2012-234405 A  (Panasonic Corp.),<br>29 November 2012 (29.11.2012),<br>entire text; all drawings<br>(Family: none) | 1-20 |

☒ Further documents are listed in the continuation of Box C.      ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 10 February 2016 (10.02.16) | 23 February 2016 (23.02.16) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
| --- | --- |

Form PCT/ISA/210 (second sheet) (January 2015)

**INTERNATIONAL SEARCH REPORT**

| International application No. |
| --- |
| PCT/JP2015/083035 |

C (Continuation).　DOCUMENTS CONSIDERED TO BE RELEVANT

| Category* | Citation of document, with indication, where appropriate, of the relevant passages | Relevant to claim No. |
| --- | --- | --- |
| A | JP 2012-243043 A (Panasonic Corp.), 10 December 2012 (10.12.2012), entire text; all drawings (Family: none) | 1-20 |
| A | JP 2008-284165 A (Autonetworks Technologies, Ltd.), 27 November 2008 (27.11.2008), entire text; all drawings (Family: none) | 1-20 |
| A | JP 2013-37506 A (Dainippon Printing Co., Ltd.), 21 February 2013 (21.02.2013), entire text; all drawings (Family: none) | 1-20 |
| A | JP 2014-516593 A (Orsan Medical Technologies, Ltd.), 17 July 2014 (17.07.2014), entire text; all drawings & US 2012/0203121 A1 & WO 2012/107842 A2 & EP 2672883 A2 & CN 103517670 A | 1-20 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006320735 A

**Non-patent literature cited in the description**

- Brain Science, Now and Here; Forefront of Solution Business Enabled. Visualization Technology of Brain Functions. Japan Economic Newspaper, 17 November 2014

- Practical Use of Optical Topographical Technology. Hitachi High-Technologies Corp, 17 November 2014